# EUROPEAN PATENT SPECIFICATION

(11) **EP 1 809 273 B1**
(45) Date of publication and mention of the grant of the patent: **14.04.2010**
(21) Application number: 05810288.0
(22) Date of filing: 15.09.2005
(51) Int. Cl.: A61K 31/27, A61P 25/08

(54) **USE OF 2-PHENYL-1,2-ETHANEDIOL-(DI)CARBAMATES FOR TREATING EPILEPTOGENESIS**
VERWENDUNG VON 2-PHENYL-1,2-ETHANEDIOL-(DI)CARBAMATEN ZUR BEHANDLUNG VON EPILEPTOGENESE
UTILISATION DE 2-PHENYL-1,2-ETHANEDIOL-(DI)CARBAMATES POUR LE TRAITEMENT DE L'EPILEPTOGENESE

(30) Priority: 16.09.2004 US 610276 P; 12.07.2005 US 698625 P; 11.08.2005 US 707242 P
(43) Date of publication of application: 25.07.2007
(73) Proprietor: Janssen Pharmaceutica NV, 2340 Beerse (BE)
(72) Inventor: CHOI, Yong Moon, Pine Brook, New Jersey 07058 (US); GORDON, Robert, Robbinsville, New Jersey 08691 (US); NOVAK, Gerald P., Skillman, New Jersey 08558 (US); PLATA-SALAMAN, Carlos R., Zionsville, Indiana 46077 (US); TWYMAN, Roy E, Doylestown, Pennsylvania 18901 (US); WHITE, H. Steve, Salt Lake City, Utah 84124 (US); ZHAO, Boyu, Lansdale, Pennsylvania 19446 (US)
(74) Representative: Fisher, Adrian John
(86) International application number: PCT/US2005/032861
(87) International publication number: WO 2006/033947

(56) References cited:
- WO-A-02/067921
- WO-A-02/067924
- US-A- 5 698 588
- US-A- 5 854 283
- US-A- 6 103 759
- US-A1- 2002 099 418
- US-A1- 2002 156 070

## Description

### BACKGROUND OF THE INVENTION

### FIELD OF THE INVENTION

This invention relates generally to the fields of pharmacology, neurology and psychiatry. In particular, the present invention provides compounds for use in treating, preventing, reversing, arresting or inhibiting epileptogenesis as defined in claim 1.

### DESCRIPTION OF THE RELATED ART

Injuries or trauma of various kinds to the central nervous system (CNS) or the peripheral nervous system (PNS) can produce profound and long-lasting neurological and psychiatric symptoms and disorders. One common mechanism for the production of these effects is the induction of seizure activity or seizure-like phenomena in the CNS or in the nerves and ganglia of the PNS. Symptomatic of paroxysmal disturbances in CNS or PNS electrical activity, seizures or seizure-like neurological mechanisms are believed to underlie many of the pathological phenomena in a wide variety of neurological and psychiatric disorders.

One serious neurological condition characterized by seizures is epilepsy. Epilepsy is a common but devastating disorder affecting more than two and a half million people in the United States alone. Epilepsy describes a condition in which a person has recurrent seizures due to a chronic, underlying process. Epilepsy refers to a clinical phenomenon rather than a single disease entity, since there are many forms and causes of epilepsy. Using a definition of epilepsy as two or more unprovoked seizures, the incidence of epilepsy is estimated at approximately 0.3 to 0.5 percent in different populations throughout the world, with the prevalence of epilepsy estimated at 5 to 10 people per 1000.

On the basis of clinical and encephalographic phenomenon, four subdivisions of epilepsy are recognized: grand mal epilepsy (with subgroups: generalized, focal, jacksonian), petit mal epilepsy, psychomotor or temporal lobe epilepsy (with subgroups: psychomotor proper or tonic with adversive or torsion movements or masticatory phenomenon, automatic with amnesia, or sensory with hallucinations or dream states) and autonomic or diencephalic epilepsy (with flushing, pallor, tachycardia, hypertension, perspiration or other visceral symptoms).

While epilepsy is one of the foremost examples of a seizure-related disorder, a wide variety of neurological and psychiatric symptoms and disorders may have, as their etiology, seizures or related seizure-like neurological phenomenon. In simple terms, a seizure or a related seizure-like neurological phenomenon is a single discrete clinical event caused by an excessive electrical discharge from a collection of neurons or a seizure susceptible group of neurons through a process termed "ictogenesis". As such, ictogenic seizures may be merely the symptom of a disease. However, epilepsy and other analogous seizure-related disorders are dynamic and often progressive diseases, with a maturation process characterized by a complex, and poorly understood sequence of pathological transformations.

The development and maturation of such changes is the process of "epileptogenesis," whereby the larger collection of neurons that is the normal brain is altered and subsequently becomes susceptible to abnormal, spontaneous, sudden, recurrent, excessive electrical discharges, i.e., seizures. The maturation of the epileptogenic process results in the development of an "epileptogenic focus," whereby the collections of abnormally discharging neurons or neurons susceptible to seizures form localized groups or "epileptogenic zones" interspersed throughout the cortical tissue. The epileptogenic zones are biochemically inter-connected such that an abnormal ictogenic discharge is able to cascade from zone to zone.

As epileptogenesis progresses, the involved areas of the nervous system become more susceptible to a seizure and it becomes easier for a seizure to be triggered, resulting in progressively debilitating symptoms of the seizure or seizure-related disorder.

While ictogenesis and epileptogenesis may have a common origin in certain biochemical phenomenon and common neuronal pathways in various diseases, the two processes are not identical. Ictogenesis is the initiation and propagation of a seizure in a discrete time and space, a rapid and definitive electrical/chemical event that occurs over a period of time ranging from seconds to minutes.

Comparatively, epileptogenesis is a gradual biochemical or neuronal restructuring process whereby the normal brain is transformed by ictogenic events into an epileptogenically focused brain, having neuronal circuitry that becomes sensitized and responsive to ictogenic events, making an individual increasingly susceptible to the recurrence of spontaneous, episodic, time-limited seizures, resulting in progressively debilitating symptoms of the seizure or seizure-related disorder and progressive non-responsiveness to treatment. The maturation of an "epileptogenic focus" is a slow biochemical and/or structural process that generally occur over months to years.

### Epileptogenesis is a Two Phase Process:

"Phase 1 epileptogenesis" is the initiation of the epileptogenic process prior to the first epileptic seizure or symptom of an analogous seizure-related disorder, and is often the result of some kind of injury or trauma to the brain, i.e., stroke, disease (e.g., infection such as meningitis), or trauma, such as an accidental blow to the head or a surgical procedure performed on the brain.

"Phase 2 epileptogenesis" refers to the process during which brain tissue that is already susceptible to epileptic seizures or seizure related phenomena of an analogous seizure-related disorder, becomes still more susceptible to seizures of increasing frequency and/or severity and/or becomes less responsive to treatment.

While the processes involved in epileptogenesis have not been definitively identified, some researchers believe that the up regulation of excitatory coupling between neurons, mediated by N-methyl-D-aspartate (NMDA) receptors, is involved. Other researchers implicate down regulation of inhibitory coupling between neurons, mediated by gamma-amino-butyric acid (GABA) receptors. Many other factors may be involved in this process relating to the presence, concentration or activity of NO (nitric oxide) or iron, calcium or zinc ions.

Although epileptic seizures are rarely fatal, large numbers of patients require medication to avoid the disruptive, and potentially dangerous consequences of seizures. In many cases, medication used to manage the epileptic seizures or symptoms of an analogous seizure-related disorder is required for extended periods of time, and in some cases, a patient must continue to take such prescription medication for life. Furthermore, such drugs are only effective for the management of symptoms and have side effects associated with chronic, prolonged usage.

A wide variety of drugs available for the management of epileptic seizures include older agents such as phenytoin, valproate and carbamazepine (ion channel blockers), as well as newer agents such as felbamate, gabapentin, topiramate and tiagabine. In addition, for example, β-alanine has been reported to have anti-seizure activity, NMDA inhibitory activity and GABAergic stimulatory activity, but has not been employed clinically to treat epilepsy.

Accepted drugs for the treatment of epilepsy are anticonvulsant agents or, more properly termed, anti-epileptic drugs (AEDs), wherein the term "anti-epileptic" is synonymous with "anti-seizure" or "anti-ictogenic". These drugs therapeutically suppress seizures by blocking the initiation of a single ictogenic event. But those AED's now clinically available, do not prevent the process of epileptogenesis.

In treating seizures or related symptoms of analogous seizure-rotated disorders, that is for diseases and disorders with seizure-like neurological phenomenon that may apparently be related to seizures disorders, such as mood cycling in Bipolar Disorder, impulsive behaviour in patients with Impulse Control Disorders or for seizures resulting from brain injury, some AEDs may also be therapeutically useful. However, those AED's now approved are unable to prophylactically or therapeutically prevent the initial development or progressive maturation of epileptogenesis to an epileptogenic focus that also characterizes analogous seizure-related disorders.

The poorly understood pathological mechanisms that underlie epileptogenesis certainly play a role in the development of epilepsy and analogous seizure-related disorders under a variety of clinical circumstances including spontaneous development or as a result of injury or trauma of many kinds to the central or peripheral nervous system.

Current epilepsy treatment is focused on suppressing seizure activity by administering AEDs after overt clinical epilepsy has developed. Although AEDs have positive effects in suppressing seizures, those now available have been universally unsuccessful in preventing epileptogenesis, i.e., the initial development or progression and worsening of epilepsy and other related seizure-like diseases. Even pretreatment with AEDs does not prevent the development of epilepsy after injury or trauma to the nervous system. Moreover, if therapy with AEDs is discontinued, the seizures typically recur and, in unfortunate instances, worsen with time. Currently, there is no clinically available method for treating, preventing, reversing, arresting or inhibiting the onset and/or progression of epilepsy or other seizure disorders or the many analogous seizure-related disorders.

In addition, it is also believed that similar neurological mechanisms corresponding to epileptogenesis may be involved in the evolution and development of many seizure-related disorders clinically analogous to epilepsy that do not appear to be overtly "epileptic," such as the initial development and progressive worsening observed in the mature disease state in Bipolar Disorder, Impulse Control Disorders, Obsessive-Compulsive disorders, Schizoaffective disorders, Substance Abuse or Addictive Disorders and many other psychiatric and neurological disorders.

Thus, despite the numerous drugs available for the treatment of epilepsy (i.e., through suppression of ictus epilepticus, i.e., the convulsions associated with epileptic seizures) and other analogous seizure-related disorders, there are no generally accepted drugs for treating, preventing, reversing, arresting or inhibiting the underlying process of epileptogenesis that may be etiologic in many devastating neurological and psychiatric disorders such as epilepsy and analogous seizure-related disorders including Bipolar Disorder.

Currently, there are no known methods of inhibiting the epileptogenic process to prevent the development of epilepsy or other analogous seizure-related disorders in patients who have not yet clinically shown symptoms thereof, but who unknowingly have the disease or are at risk of developing the disease. In addition, there are no known methods to prevent the development of or reverse the process of epileptogenesis, thus converting the collections of neurons in an epileptogenic zone which have been the source of or are susceptible or are capable of participating in seizure activity into nerve tissue that does not exhibit abnormal, spontaneous, sudden, recurrent or excessive electrical discharges or is not susceptible to or capable of such seizure activity. Furthermore, there are no approved or unapproved medications recognized as having such anti-epileptogenic properties, i.e., truly anti-epileptogenic drugs (AEGDs) (See, Schmidt, D. and Rogawski, M. A., Epilepsy Research, 2002, 50; 71-78).

Thus, there is a great need to develop safe and effective drugs or AEDs and methods of treatment that effectively; treat, prevent, arrest, inhibit and reverse epileptogenesis in seizure-related neurological and/or psychiatric disorders.

### SUMMARY OF THE INVENTION

This invention relates to compounds for use in the treatment and/or prevention, arrest, inhibition and reversal of epileptogenesis in a patient at risk for the development of a seizure disorder or an analogous seizure-related disorder.

This invention is based, in part, on the previously unknown property of the carbamate compounds of the invention. These compounds are effective AEDs and can suppress epileptic seizures and, in addition, are powerfully anti-epileptogenic and can prevent the initial development and maturation of the pathological changes in the nervous system that allow seizures and related phenomena to occur and/or spread and may be able to reverse those changes. Thus, the carbamate compounds of the present invention, as used in this invention, are true anti-epileptogenic drugs (AEGDs) and have properties that are distinctly different from and not possessed by any presently approved AED medication.

The compounds used in the present invention are of Formula 1 or Formula 2: 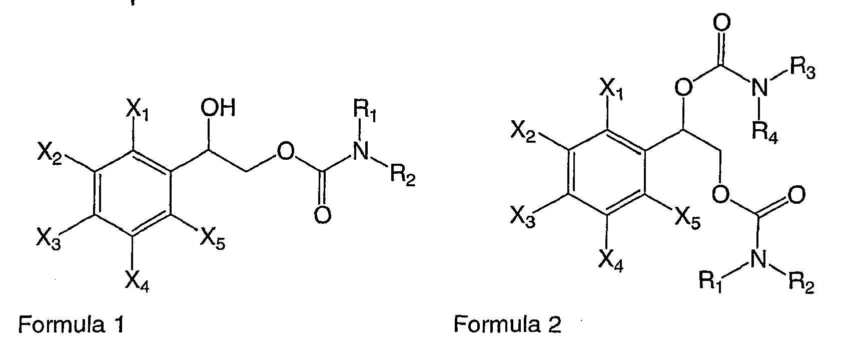 or a pharmaceutically acceptable salt or ester form thereof, wherein R₁, R₂, R₃ and R₄ are independently hydrogen or C₁-C₄ alkyl, wherein C₁₋C₄alkyl is substituted or unsubstituted with phenyl, and wherein phenyl is substituted or unsubstituted with up to five substituents independently selected from; halogen, C₁-C₄ alkyl, C₁-C₄ alkoxy, amino, wherein amino is optionally mono or disubstituted with C₁-C₄ alkyl, nitro or cyano; and X₁, X₂, X₃, X₄ and X₅ are independently hydrogen, fluorine, chlorine, bromine or iodine. Embodiments of the present invention include a compound of Formula 1 or Formula 2 wherein X₁, X₂, X₃, X₄ and X₅ are independently selected from hydrogen, fluorine, chlorine, bromine or iodine.

In certain embodiments, X₁, X₂, X₃, X₄ and X₅ are independently selected from hydrogen or chlorine. In other embodiments, X₁ is selected from fluorine, chlorine, bromine or iodine. In another embodiment, X₁ is chlorine, and X₂, X₃, X₄ and X₅ are hydrogen. In another embodiment, R₁, R₂, R₃ and R₄ are hydrogen.

The present invention provides enantiomers of Formula 1 or Formula 2 for use in treating epileptogenesis in a subject in need thereof as defined in claim 1. In certain embodiments, a compound of Formula 1 or Formula 2 will be in the form of a single enantiomer thereof. In other embodiments, a compound of Formula 1 or Formula 2 will be in the form of an enantiomeric mixture in which one enantiomer predominates with respect to another enantiomer.

In another aspect, one enantiomer predominates in a range of from about 90% or greater. In a further aspect, one enantiomer predominates in a range of from about 98% or greater.

In one compound of Formula 1 or Formula 2, R₁, R₂, R₃ and R₄ may be independently selected from hydrogen or C₁-C₄ alkyl; and X₁, X₂, X₃, X₄ and X₅ are independently selected from hydrogen, fluorine, chlorine, bromine or iodine.

Before prophylactic or therapeutic administration of the composition of the invention to the subject, a determination will be made as to whether or not the subject is at a high risk for the development of epilepsy or seizure-related disorders.

In certain embodiments of the present invention, a prophylactically or therapeutically effective amount of a compound of Formula 1 or Formula 2 for the treatment of epileptogenesis is in a range of from about 400 mg/day to about 3000 mg/day (about 5.7 mg/Kg/day to about 43.0 mg/Kg/day in a 70 kg human). Thus the pharmaceutical compounds and compositions of the invention may be administered at a dosage of from about 5.7 to about 43.0 mg/kg/day (400-3000 mg/day in a 70 kg human), preferably from about 6.4 to about 35.7 mg/kg/day (450-2500 mg/day in a 70 kg human), more preferably from about 7.1 to about 28.6 mg/kg/day (500-2000 mg/day in a 70 kg human), or even more preferably from about 7.9 to about 21.4 mg/kg/day (550-1500 mg/day in a 70 kg human) or most preferably from about 8.6 to about 17.1 mg/kg/day (600-1200 mg/day in a 70 kg human). The dosages, however, may be varied depending the individual characteristics and tolerances of the subject and the on the precise nature of the condition being treated.

In certain embodiments, a prophylactically or therapeutically effective amount of a pharmaceutical composition for preventing, treating, reversing, arresting or inhibiting epileptogenesis comprising one or more of the enantiomers of a compound of Formula 1 or Formula 2 includes a pharmaceutically acceptable salt or ester thereof in admixture with a pharmaceutically acceptable carrier or excipient, whereby such a composition is administered to the subject in need of treatment with an AEGD. Pharmaceutical compositions comprising at least one compound having Formula 1 or Formula 2 and one or more pharmaceutically acceptably excipients are administered to a subject in need thereof.

In another aspect, the subject or patient will be determined to be at risk for developing epilepsy or an analogous seizure-related disorder at the time of administration and on this basis will be considered to be a patient in need of treatment with an AEGD.

### BRIEF DESCRIPTION OF THE FIGURES

Figure 1: is a graph that shows the effects of increasing doses of TC on the number of neurons in different areas of the hippocampus counted at 14 days after li-pilo SE. Values are expressed as the number of neuronal cell bodies in each area of interest ± S.E.M.
Figure 2: is a graph that shows the effects of increasing doses of TC on the number of neurons in different nuclei of the amygdala counted at 14 days after li-pilo SE. Values are expressed as the number of neuronal cell bodies in each area of interest ± S.E.M.
Figure 3: is a graph that shows the effects of increasing doses of TC on the number of neurons in different nuclei of the thalamus counted at 14 days after li-pilo SE. Values are expressed as the number of neuronal cell bodies in each area of interest ± S.E.M.
Figure 4: is a graph that shows the effects of increasing doses of TC on the number of neurons in different areas of the cortex counted at 14 days after li-pilo SE. Values are expressed as the number of neuronal cell bodies in each area of interest ± S.E.M.
Figure 5: is a graph that shows the effects of increasing doses of TC on the latency to the first spontaneous seizure. Values are expressed as the mean latency in days for each group ± S.E.M.
Figure 6: is a graph that shows the effects of increasing doses of TC on the frequency of spontaneous seizures video-recorded over a 4 weeks period. Values are expressed as the mean number of seizures ± S.E.M. The total represents the total number of seizures observed during the 4 weeks of video-recording and the mean represents the mean number of seizures per week. The Anova test demonstrated an effect of the treatment on the total number of seizures (p=0.045) and the mean number of seizures per week (p=0.045)
Figure 7: shows the total number of seizures video-recorded over four weeks plotted according to the latency to the first spontaneous seizure (SL = short latency, LL = long latency). Values are expressed as the mean number of seizures for each subgroup ± S.E.M. The ANOVA test did not show any significant effect of the treatment.
Figure 8: shows the correlation between the latency to the first spontaneous seizure and the total number of seizures observed during the four following weeks.

### DETAILED DESCRIPTION OF THE INVENTION

The present invention provides 2-phenyl-1, 2-ethanediol monocarbamates and dicarbamates for use in the treatment and/or prevention of epileptogenesis as defined in claim 1.

### The Carbamate Compounds of the Invention

Representative carbamate compounds according to the present invention include those having Formula 1 or Formula 2: 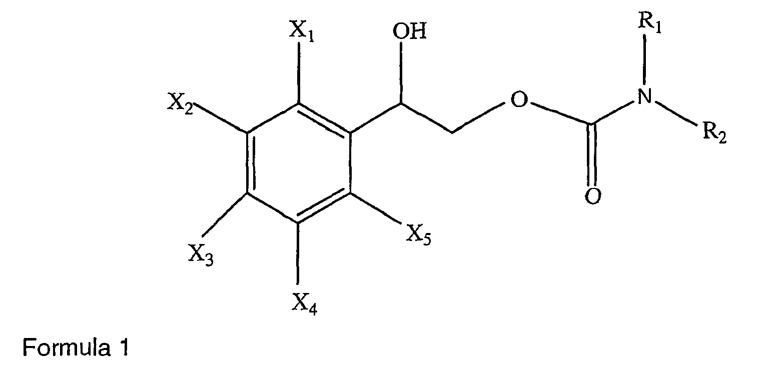 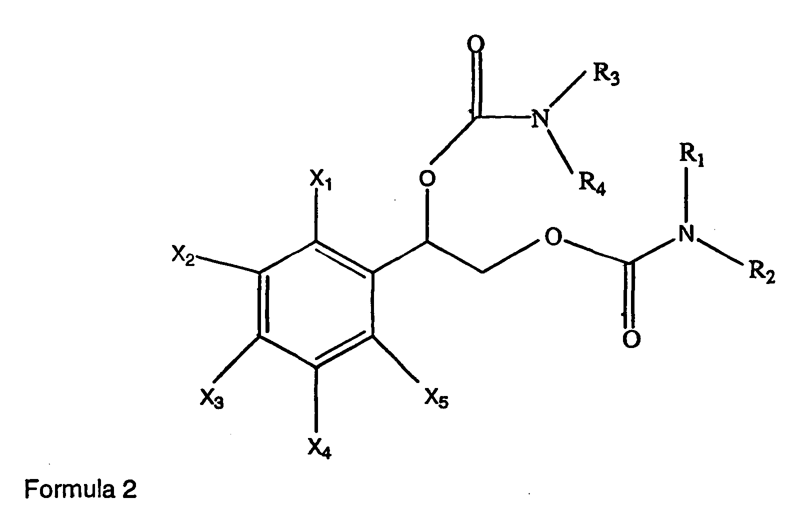 wherein:
R₁, R₂, R₃, and R₄ are, independently, hydrogen or C₁-C₄ alkyl and X₁, X₂, X₃, X₄, and X₅ are, independently, hydrogen, fluorine, chlorine, bromine or iodine.
   "C₁-C₄ alkyl" as used herein refers to substituted or unsubstituted aliphatic hydrocarbons having from 1 to 4 carbon atoms. Specifically included within the definition of "alkyl" are those aliphatic hydrocarbons that are optionally substituted. In a preferred embodiment of the present invention, the C₁-C₄ alkyl is either unsubstituted or substituted with phenyl.

The term "phenyl", as used herein, whether used alone -or as part of another group, is defined as a substituted or unsubstituted aromatic hydrocarbon ring group having 6 carbon atoms. Specifically included within the definition of "phenyl" are those phenyl groups that are optionally substituted. For example, in a preferred embodiment of the present invention, the, "phenyl" group is either unsubstituted or substituted with halogen, C₁-C₄ alkyl, C₁-C₄ alkoxy, amino, nitro, or cyano.

In a preferred embodiment of the present invention, X₁ is fluorine, chlorine, bromine or iodine and X₂, X₃, X₄, and X₅ are hydrogen.

In another preferred embodiment of the present invention, X₁, X₂, X₃, X₄, and X₅ are, independently, chlorine or hydrogen.

In another preferred embodiment of the present invention, R₁, R₂, R₃, and R₄ are all hydrogen.

It is understood that substituents and substitution patterns on the compounds of the present invention can be selected by one of ordinary skill in the art to provide compounds that are chemically stable and that can be readily synthesized by techniques known in the art as well as the methods provided herein.

Representative 2-phenyl-1, 2-ethanediol monocarbomates and dicarbamates include, for example, the following compounds:

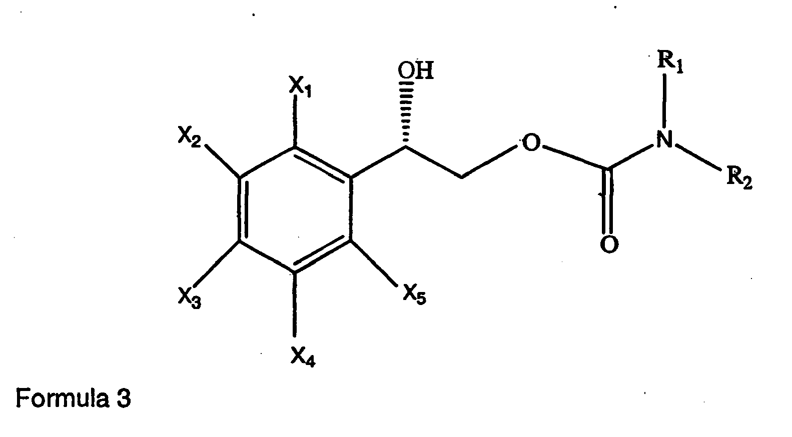

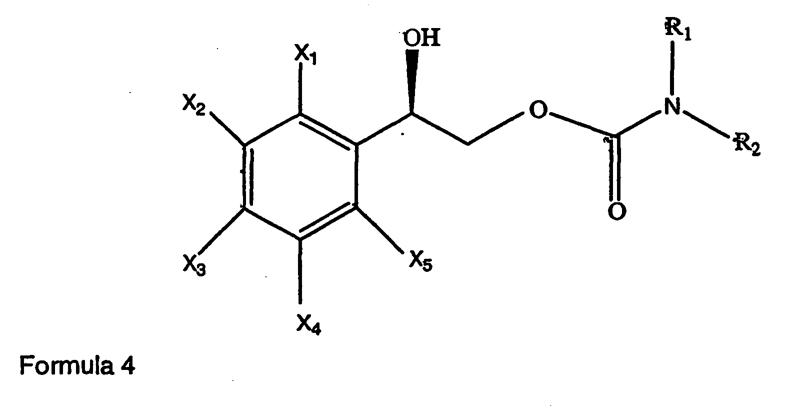

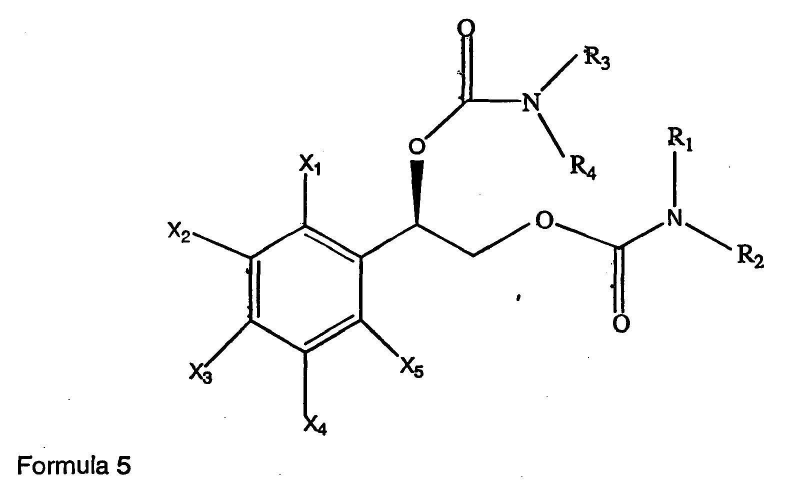

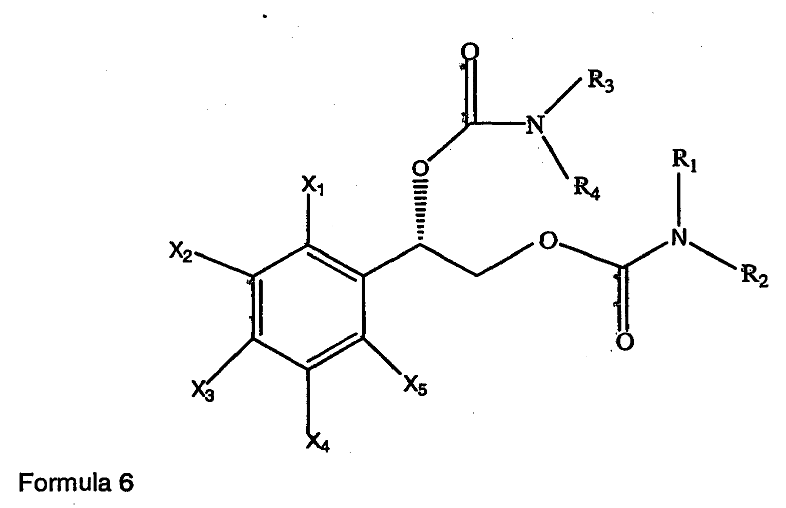

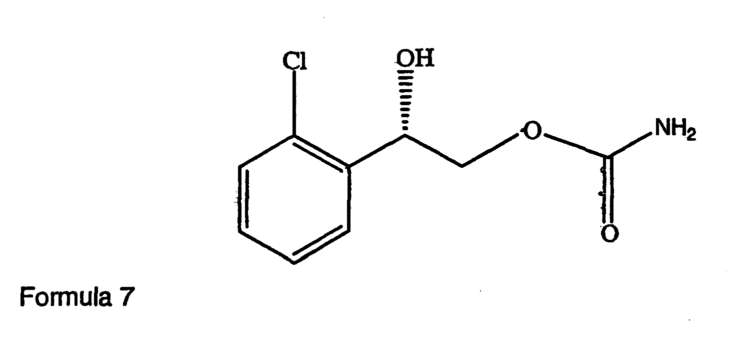

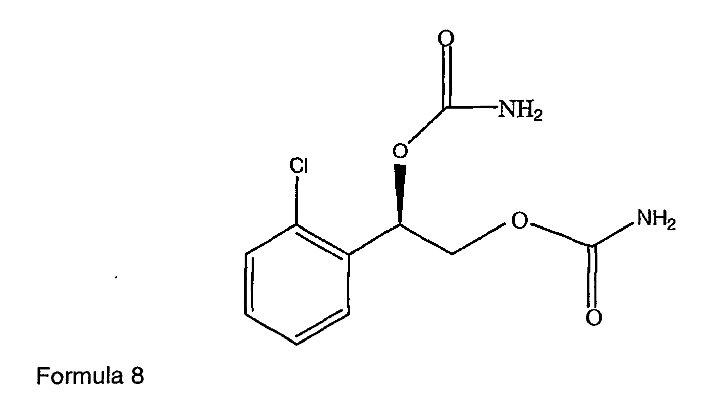

Suitable methods for synthesizing and purifying carbamate compounds, including carbamate enantiomers, used in the methods of the present invention are well known to those skilled in the art. For example, pure enantiomeric forms and enantiomeric mixtures of 2-phenyl-1, 2-ethanediol monocarbomates and dicarbamates are described in United States Patent Numbers 5,854,283, 5,698,588, and 6,103,759, the disclosures of which are herein incorporated by reference in their entirety.

The compounds for use in the present invention includes isolated enantiomers of Formula 1 or Formula 2.

In one preferred embodiment, a pharmaceutical composition comprising the isolated S-enantiomer of Formula 1 is used to treat epileptogenesis in a subject.

In another preferred embodiment, a pharmaceutical composition comprising the isolated R-enantiomer of Formula 2 is used to treat epileptogenesis in a subject.

In another embodiment, a pharmaceutical composition comprising the isolated S-enantiomer of Formula 1 and the isolated R-enantiomer of Formula 2 can be used to treat epileptogenesis in a subject.

The compounds for use in the present invention also includes the use of mixtures of enantiomers of Formula 1 or Formula 2. In one aspect of the present invention, one enantiomer will predominate. An enantiomer that predominates in the mixture is one that is present in the mixture in an amount greater than any of the other enantiomers present in the mixture, e.g., in an amount greater than 50%. In one aspect, one enantiomer will predominate to the extent of 90% or to the extent of 91%, 92%, 93%, 94%, 95%, 96%, 97% or 98% or greater. In one preferred embodiment, the enantiomer that predominates in a composition comprising a compound of Formula 1 is the S-enantiomer of Formula 1. In another preferred embodiment, the enantiomer that predominates in a composition comprising a compound of Formula 2 is the R-enantiomer of **Formula 2.**

In a preferred embodiment of the present invention, the enantiomer that is present as the sole enantiomer or as the predominate enantiomer in a composition of the present invention is represented by Formula 3 or Formula 5, wherein X₁, X₂, X₃, X₄, X₅, R₁, R₂, R₃, and R₄ are defined as above, or by **Formula 7 or Formula 8.**

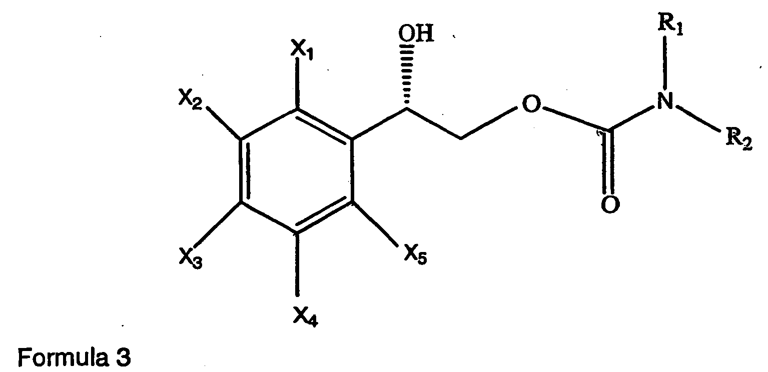

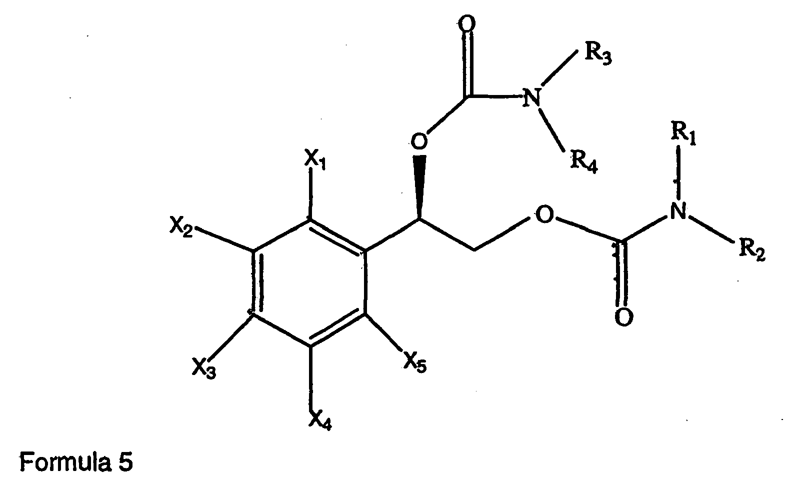

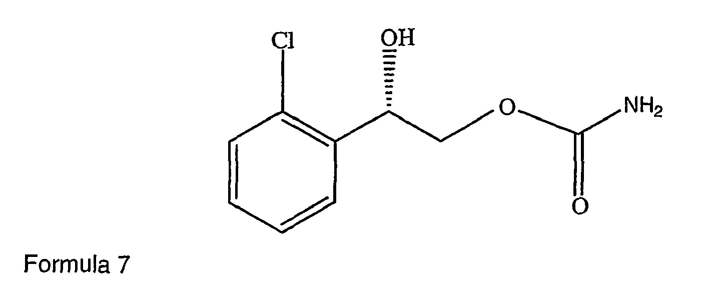

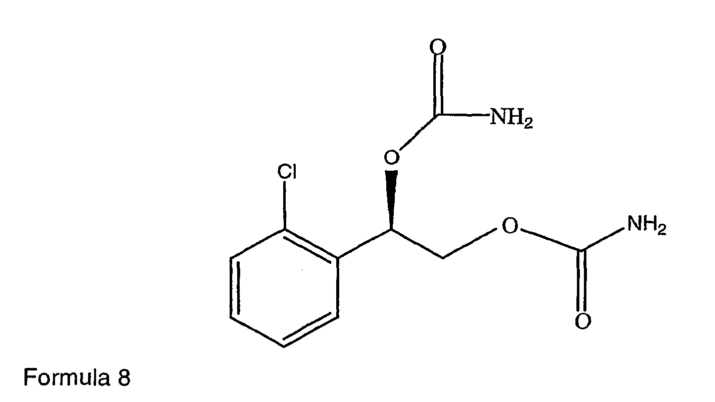

A carbamate enantiomer of Formula 1 or Formula 2 contains an asymmetric chiral carbon at the benzylic position, which is the aliphatic carbon adjacent to the phenyl ring.

An enantiomer that is isolated is one that is substantially free of the corresponding enantiomer. Thus, an isolated enantiomer refers to a compound that is separated via separation techniques or prepared free of the corresponding enantiomer. "Substantially free," as used herein, means that the compound is made up of a significantly greater proportion of one enantiomer. In preferred embodiments, the compound includes at least about 90% by weight of a preferred enantiomer.

In other embodiments of the invention, the compound includes at least about 99% by weight of a preferred enantiomer. Preferred enantiomers can be isolated from racemic mixtures by any method known to those skilled in the art, including high performance liquid chromatography (HPLC) and the formation and crystallization of chiral salts, or preferred enantiomers can be prepared by methods described herein.

Methods for the preparation of preferred enantiomers would be known to one of skill in the art and are described, for example, in Jacques, et al., Enantiomers, Racemates and Resolutions (Wiley Interscience, New York, 1981); Wilen, S.H., et al., Tetrahedron 33:2725 (1977); Eliel, E.L. Stereochemistry of Carbon Compounds (McGraw-Hill, NY, 1962); and Wilen, S.H. Tables of Resolving Agents and Optical Resolutions p. 268 (E.L. Eliel, Ed., Univ. of Notre Dame Press, Notre Dame, IN 1972).

Additionally, compounds of the present invention can be prepared as described in United States Patent Number 3,265,728 3,313,692 and the previously referenced United States Patent Numbers 5,854,283, 5,698,588, and 6,103,759.

### The Treatment of Epileptogenesis

By using the compounds and compositions of this invention it is possible to suppress, control and prevent the process of epileptogenesis that results in the worsening, clinical progression or increasing resistance to treatment of epilepsy and related seizure disorders or to the de-novo initiation of these disorders and their symptoms as a result of some form of injury or trauma to the nervous system.

The subject or patient in need of treatment is be a subject who has not shown the symptoms of epilepsy, i.e., seizures or convulsions prior to the time of administration. The subject or patient will be determined to be at risk for developing epilepsy and on this basis will be considered to be a patient in need of treatment with an AEGD.

By suppressing the process of epileptogenesis the development of a seizure disorder or a related disorder can be prevented in a subject who has sustained some form of injury or damage to the nervous system or who is otherwise at risk.

### Epilepsy

The term epilepsy refers to a disorder of brain function characterized by the periodic and unpredictable occurrence of seizures (See, The Treatment of Epilepsy, Principles & Practice, Third Edition, Elaine Wyllie, M.D. Editor, Lippincott Williams & Wilkins, 2001; Goodman & Gilman's The Pharmacological Basis of Therapeutics, 9th edition, 1996). Seizures that occur without evident provocation are classified as epileptic. Epilepsy may be idiopathic or may be related to some kind of injury, malformation or damage to the central nervous at any stage of life. A subject is typically considered to suffer from epilepsy upon experiencing two or more seizures that occur more than 24 hours apart.

Clinically, an epileptic seizure results from a sudden and abnormal electrical discharge originating from a collection of interconnected neurons in the brain or elsewhere in the nervous system. Depending on the type of epilepsy involved, the resulting nerve cell activity may be manifested by a wide variety of clinical symptoms such as uncontrollable motor movements, changes in the patient's level of consciousness and the like. Epilepsy and epileptic seizures and syndromes may be classified in a variety of ways (See, The Treatment of Epilepsy, Principles & Practice, Third Edition, Elaine Wyllie, M.D. Editor, Lippincott Williams & Wilkins, 2001). However, as used herein the terms; "epilepsy", "epileptic seizures" and "epileptic syndromes" are meant to include all known types of epileptic seizures and syndromes including; partial seizures, including simple, complex and partial seizures evolving to generalized tonic-clonic convulsions and generalized seizures, both convulsive and nonconvulsive and unclassified epileptic seizures.

### The Epileptogenic Process

The epileptogenic process generally consists of two phases. The first epileptogenic stage is known as the initial insult or injury stage. The initial insult or injury is commonly a brain-damaging injury caused by one or more of a multitude of possible factors including, for example, traumatic brain injury, including blunt and penetrating head trauma or a neurosurgical procedure; CNS infection, such as, for example, bacterial meningitis, viral encephalitis, bacterial cerebral abscess or neurocysticercosis); cerebrovascular disease (such as stroke or brain tumor including, for example, malignant gliomas; neurosurgery (such as for example craniotomies) and status epilepticus.

In some instances, the initial insult will be a result of developmental problems before birth (such as, but not limited to, birth asphyxia, intracranial trauma during birth, metabolic disturbances or congenital malformations of the brain) or as the result of genetic determinants.

The second epileptogenic stage is known as the latency stage.

The second epileptogenic stage further includes the process of neuronal restructuring, which is characterized by recurrent seizures (e.g. symptomatic epilepsy) or by symptoms shown in analogous seizure-related disorders. The epileptogenic process can also be observed among persons actually suffering from epilepsy or analogous seizure-related disorders. The seizures experienced by persons suffering from epilepsy are themselves epileptogenic in that they tend to make the occurrence of subsequent seizures more likely or extend the area of nervous tissue that is subject to seizure activity or make the seizure disorder more resistant to treatment. The consequences of this process, for a patient who has a seizure disorder, is that the seizures tend to become more frequent and more severe and often more resistant to treatment with conventional AED's

In a similar manner, the related seizure-like response in neurological or psychiatric disorders analogous to epilepsy may become increasingly severe over time or resistant to treatment as the disorder matures.

In certain embodiments, phase 1 epileptogenesis can be initiated by factors other than those listed above, such as by the ingestion of compounds with epileptogenic potential, e.g., psychotropic medications such as, for example, tricyclic antidepressants, clozapine, and lithium and the like. The compounds of the present invention are also intended to treat, prevent, arrest, inhibit or reverse the development of epileptogenesis which has been initiated by factors which tend to increase the potential for a subject to become epileptogenic.

Therefore, in treating epileptogenesis, the compounds of the invention can forestall the development of seizures, particularly epileptic seizures. Such compounds therefore can be used to reduce the risk of developing epilepsy, arrest the development of epilepsy (particularly, the development of collections of neurons which are the source of or are susceptible to ictogenic seizure), inhibit the development and maturation of epilepsy (particularly, the development of epileptogenic zones and epileptogenic focus).

In addition, by treating, preventing, inhibiting, arresting or reversing epileptogenesis according to the present invention, the development or progression of analogous neurological and/or psychiatric disorders whose etiology is partly or wholly based on a seizure like mechanism of action will be treated, prevented, inhibited, arrested or reversed.

In some embodiments the compounds of the present invention will be advantageously used to treat a patient who is not suffering or known to be suffering from a condition that is known in the art to be effectively treated with presently known anticonvulsant or antiepileptic (AED) medications. In these cases the decision to use the methods and compounds of the present invention would be made on the basis of determining if the patient is a "patient in need of treatment with an anti-epileptogenic drug (AEGD)" as that term is defined above.

The compounds of the present invention are for administration to a patient who has not developed epilepsy or any type of seizure disorder or an analogous seizure related disorder but who may be in a high risk group for the development of seizures or an analogous seizure related disorder because of injury or trauma to the nervous system that has occurred, including but not limited to head injury or stroke or may occur in the future, including but not limited to planned neurosurgical procedures or because of some known predisposition either biochemical or genetic or the finding of a verified biomarker of one or more of these disorders.

Thus, compositions of the present invention are directed toward treating epileptogenesis in a subject who is at risk of developing epilepsy or a seizure related disorder but does not have epilepsy or clinical evidence of seizures.

A subject who is at risk of developing epilepsy but who does not have epilepsy or other seizure disorder can be a subject who has not yet been diagnosed with epilepsy but who is at greater risk than the general population for developing epilepsy. This "greater risk" may be determined by the recognition of any factor in a subject's, or their family's medical history, physical exam or testing that is indicative of a greater than average risk for developing epilepsy. Therefore this determination that a patient may be at a "greater risk" by any available means can be used to determine whether the patient should be treated with the compounds of the present invention.

Patients who are at greater risk would also include but is not limited to those who have not suffered damage or injury to their central nervous system but have a high likelihood of such damage or injury either because of their medical condition or their environment. This would include, but not be limited to; patients with a history of Transient Ischemic Attacks (TIA's) or known carotid artery stenosis or simply known significant arteriosclerosis as well as patients about to undergo a neurosurgical procedure. In addition, individuals likely to suffer neurological damage due to war or sports injury could be prophylactically administered compounds of the invention; this would include soldiers in combat or athletes in violent contact sports such as boxing.

Accordingly, in an exemplary embodiments, subjects who may benefit from treatment by the compounds of this invention can be identified using accepted screening methods to determine risk factors associated with epileptogenesis, epilepsy or other seizure disorders.

A determination that a subject has, or may be at risk for developing, epilepsy, another seizure disorder would also include, for example, a medical evaluation that includes a thorough history, a physical examination, and a series of relevant bloods tests. It can also include an electroencephalogram (EEG), computer tomography (CT), magnetic resonance imaging (MRI) or positron emission tomography (PET). A determination of an increased risk of developing epilepsy may also be made by means of genetic testing, including gene expression profiling or proteomics techniques. (See, Schmidt, D. Rogawski, M. A. Epilepsy Research 50; 71-78 (2002), and Loscher, W, Schmidt D. Epilepsy Research 50; 3-16 (2002))

These screening methods include, for example, conventional medical work-ups to determine risk factors that may be associated with epileptogenesis including but not limited to:, for example, head trauma, either closed or penetrating, neurosurgical procedures, CNS infections, bacterial or viral, Trigeminal Neuralgia, cerebrovascular disease, including but not limited to, stroke or a history of TIA's, brain tumors, brain edema, cysticercosis, porphyria, metabolic encephalopathy, drug withdrawal including but not limited to sedative-hypnotic or alcohol withdrawal, abnormal perinatal history including anoxia at birth or birth injury of any kind, cerebral palsy, learning disabilities, hyperactivity, history of febrile convulsions, history of status epilepticus, family history of epilepsy or any a seizure related disorder, inflammatory disease of the brain or blood vessels including lupus, drug intoxication, either direct or by placental transfer, including but not limited to cocaine and methamphetamine toxicity, parental consanguinity, and treatment with medications that lower seizure threshold including psychotropic medications such as antidepressant or anti psychotic medications.

In some embodiments the compounds of the present invention would be used for the manufacture of a medicament for the purpose of treating a patient in need of treatment with an anti-epileptogenic drug (AEGD). This would include the manufacture of a medicament for the purpose of treating a patient is at risk of developing; epilepsy, a seizure disorder or epilepsy related seizure like neurological phenomenon or seizure related disorder, as defined above, or any disorder in which the patient's present clinical condition or prognosis could benefit from the suppression or inhibition of the process of epileptogenesis to prevent the extension, worsening or increased resistance to treatment of any neurological or psychiatric disorder.

The determination of which patients may benefit from treatment with an AEGD in patients who have no clinical signs or symptoms of epilepsy or other seizure disorder may be based on a variety of "surrogate markers" or "biomarkers". Such biomarkers would include but not be limited to gene or protein expression profiles in tissue, blood or CSF or the presence of genetic markers such as SNP's.

As used herein, the terms "surrogate marker" and "biomarker" are used interchangeably and refer to any anatomical, biochemical, structural, electrical, genetic or chemical indicator or marker that can be reliably correlated with the present existence or future development of epilepsy or a seizure disorder. In some instances, brain-imaging techniques, such as computer tomography (CT), magnetic resonance imaging (MRI) or positron emission tomography (PET), or other neurological imaging techniques can be used to determine whether a subject is at risk for developing one of the above disorders.

Examples of suitable biomarkers for the methods of this invention include, but are not limited to: the determination by MRI, CT or other imaging techniques, of sclerosis, atrophy or volume loss in the hippocampus or the presence of mesial temporal sclerosis (MTS) or similar relevant anatomical pathology; the detection in the patient's blood, serum or tissues of a molecular species such as a protein or other biochemical biomarker, e.g., elevated levels of ciliary neurotrophic factor (CNTF) or elevated serum levels of a neuronal degradation product; or other evidence from surrogate markers or biomarkers that the patient is in need of treatment with an anti-epileptogenic drug, e.g. an EEG suggestive of a seizure disorder, epilepsy related seizure like neurological phenomenon or seizure related disorder.

It is expected that many more such biomarkers utilizing a wide variety of detection techniques will be developed in the future. It is intended that any such marker or indicator of the existence or possible future development of a seizure disorder or epilepsy may be used in this invention for determining the need for treatment with the compositions and methods of this invention.

In some embodiments of the present invention carbamate compounds suitable for use in the practice of this invention will be administered either singly or concomitantly with at least one or more other compounds or therapeutic agents, e.g., with other antiepileptic drugs, anticonvulsant drugs or neuroprotective drugs or electro convulsive therapy (ECT). In these embodiments, the present invention provides compositions to treat, prevent or reverse epileptogenesis

As used herein, the term "concomitant administration" or "combination administration" of a compound, therapeutic agent or known drug with a compound of the present invention means administration of the drug and the one or more compounds at such time that both the known drug and the compound will have a therapeutic effect. In some cases this therapeutic effect will be synergistic. Such concomitant administration can involve concurrent (i.e. at the same time), prior, or subsequent administration of the drug with respect to the administration of a compound of the present invention. A person of ordinary skill in the art would have no difficulty determining the appropriate timing, sequence and dosages of administration for particular drugs and compositions of the present invention.

The said one or more other compounds or therapeutic agents may be selected from compounds that have one or more of the following properties: antioxidant activity; NMDA receptor antagonist activity, augmentation of endogenous GABA inhibition; NO synthase inhibitor activity; iron binding ability, e.g., an iron chelator; calcium binding ability, e.g., a Ca (II) chelator; zinc binding ability, e.g., a Zn (II) chelator; the ability to effectively block sodium or calcium ion channels, or to open potassium or chloride ion channels in the CNS of a patient, including known AEDs or are therapeutic agents useful in the treatment of Substance Abuse and addiction, including but not limited to, methadone, disulfiram, bupropion, antipsychotics, antidepressants, benzodiazepines, buspirone, naloxone or naltrexone.

In some preferred embodiments, the one or more other compounds or therapeutic agents would antagonize NMDA receptors by binding to the NMDA receptors (e.g., by binding to the glycine binding site of the NMDA receptors) and/or the agent would augment GABA inhibition by decreasing glial GABA uptake.

In addition the said one or more other compounds or therapeutic agents may be any agent known to suppress seizure activity even if that compound is not known to inhibit epileptogenesis. Such agents would include but not be limited to any effective AED or anti-convulsant known to one of skill in the art or discovered in the future, for example suitable agents include, but are not limited to; carbamazepine, clobazam, clonazepam, ethosuximide, felbamate, gabapentin, lamotigine, levetiracetam, oxcarbazepine, phenobarbital, phenytoin, pregabalin, primidone, retigabine, talampanel, tiagabine, topiramate, valproate, vigabatrin, zonisamide, benzodiazepines, barbiturates or sedative hypnotics.

### Definitions

As used herein, the term "epileptogenesis" means the biochemical, genetic, histological or other structural or functional processes or changes that make nervous tissue, including the central nervous system (CNS) susceptible to recurrent, spontaneous seizures. In addition, the term "epileptogenesis" is also used herein in a broader sense to refer to the changes and processes that contribute to the clinical progression observed in patients with epilepsy or other seizure disorder or an analogous seizure related disorder including but not limited to; the worsening or progression of the disorder and it's symptoms or the development of "pharmacoresistance," in which the disorder becomes more difficult to treat as a result of neurobiological changes which result in reduced drug sensitivity or the recruitment by the process of epileptogenesis of non seizure prone nervous tissue.

The term "inhibition of epileptogenesis," as used herein, refers to preventing, slowing, halting, or reversing the process of epileptogenesis.

The term "anti-epileptogenic agent or drug" (AEGD), as used herein, refers to an agent that is capable of inhibiting epileptogenesis when the agent is administered to a subject in need thereof.

The term "convulsive disorder," as used herein, refers to a disorder in a subject in which the subject suffers from convulsions, e.g., convulsions due to epileptic seizure. Convulsive disorders include, but are not limited to, epilepsy and non-epileptic convulsions, e.g., convulsions due to administration of a convulsive agent or toxin to the subject.

As used herein, the term "subject" or "patient" is a human being who has not yet shown the symptoms of epilepsy but who may be in a high risk group.

As used herein, the term "a subject in need of treatment with an AEGD" would include an individual who does not have epilepsy but who may be in a high-risk group for the development of seizures or a seizure related disorder because of injury or trauma to the central (CNS) or peripheral nervous system (PNS). An individual or patient is considered to be at a high risk for the development of such seizures or seizure-related disorders because of injury or trauma to the CNS or PNS, because of some known biochemical or genetic predisposition to epilepsy or analogous seizure-related disorder, or because a verified biomarker or surrogate marker of one or more of these disorders has been discovered.

The term "a subject in need of treatment with an AEGD" would also include any individual whose clinical condition or prognosis could benefit from treatment with an AEGD. This would include, but is not limited to, any individual determined to be at an increased risk of developing epilepsy, a seizure disorder or epilepsy related seizure like neurological phenomenon or seizure related disorder as defined above, due to any predisposing factor. Predisposing factors include, but are not limited to: injury or trauma of any kind to the CNS or PNS; infections of the CNS, e.g., meningitis or encephalitis; anoxia; stroke, i.e., cerebro-vascular accidents (CVAs); autoimmune diseases affecting the CNS, e.g., lupus; birth injures, e.g., perinatal asphyxia; cardiac arrest; therapeutic or diagnostic vascular surgical procedures, e.g., carotid endarterectomy or cerebral angiography; heart bypass surgery; spinal cord trauma; hypotension; injury to the CNS from emboli, hyper or hypo perfusion of the CNS; hypoxia affecting the CNS; known genetic predisposition to disorders known to respond to AEGDs; space occupying lesions of the CNS; brain tumors, e.g., glioblastomas; bleeding or hemorrhage in or surrounding the CNS, e.g., intracerebral bleeds or subdural hematomas; brain edema; febrile convulsions; hyperthermia; exposure to toxic or poisonous agents; drug intoxication, e.g. cocaine; family history of seizure disorders history of status epilepticus; current treatment with medications that lower seizure threshold, e.g., lithium carbonate, thorazine or clozapine; evidence from surrogate markers or biomarkers that the patient is in need of treatment with an anti-epileptogenic drug, e.g. MRI scan showing hippocampal sclerosis or other CNS pathology, elevated serum levels of neuronal degradation products.

As used herein, unless otherwise noted, the term "epilepsy" shall mean any disorder in which a subject (preferably a human adult, child or infant) experiences one or more seizures and/or tremors. Suitable examples include, but are not limited to, epilepsy (including, but not limited to, localization-related epilepsies, generalized epilepsies, epilepsies with both generalized and local seizures, and the like), seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, and the like) and the like. The term is intended to refer to the clinical disorder regardless of type of seizure, origin of seizure, progression of seizure or underlying cause or etiology.

The term "antiepileptic drug" (AED) will be used interchangeably with the term "anticonvulsant agent," and as used herein, both terms refer to an agent capable of; treating, inhibiting or preventing seizure activity or ictogenesis when the agent is administered to a subject or patient.

As used herein, the term "a subject in need of treatment with an AED" would include an individual who is known to have the disease epilepsy or has had repeated seizures or convulsions or has shown the symptoms of an analogous seizure-related disorder regardless of the etiology of these symptoms.

As used herein, "halogen" shall mean chlorine, bromine, fluorine and iodine.

As used herein, the term "alkyl" whether used alone or as part of a substituent group, include straight and branched chains. For example, alkyl radicals include methyl, ethyl, propyl, isopropyl, butyl, isobutyl, sec-butyl, t-butyl, pentyl and the like. Unless otherwise noted, "C₁₋₄alkyl" means a carbon chain composition of 1-4 carbon atoms.

When a particular group is "substituted" (e.g., alkyl, phenyl, aryl, heteroalkyl, heteroaryl), that group may have one or more substituents, preferably from one to five substituents, more preferably from one to three substituents, most preferably from one to two substituents, independently selected from the list of substituents.

With reference to substituents, the term "independently" means that when more than one of such substituents is possible, such substituents may be the same or different from each other.

To provide a more concise description, some of the quantitative expressions given herein are not qualified with the term "abut", It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The terms "subject" or "patient" are used herein interchangeably and as used herein, refer to a human being, who has been the object of treatment, observation or experiment.

As used herein, the term "compositions" is intended to encompass a product comprising the specified ingredients in the specified amounts, as well as any product which results, directly or indirectly, from combinations of the specified ingredients in the specified amounts.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

The present invention includes within its scope prodrugs of the compounds of this invention. In general, such prodrugs will be functional derivatives of the compounds that are readily convertible in vivo into the required compound. Thus, in the methods of treatment of the present invention, the term "administering" shall encompass the treatment of the various disorders described with the compositions specifically disclosed or with a composition which may not be specifically disclosed, but which converts to the specified compound in vivo after administration to the patient. Conventional procedures for the selection and preparation of suitable prodrug derivatives are described, for example, in "Design of Prodrugs", ed. H. Bundgaard, Elsevier, 1985.

For use in medicine, the salts of the compounds of this invention refer to non-toxic "pharmaceutically acceptable salts." Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptably acid such as hydrochloric acid, sulfuric acid, fumaric acid, maleic acid, succinic acid, acetic acid, benzoic acid, citric acid, tartaric acid, carbonic acid or phosphoric acid.

Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following; acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparations of pharmaceutically acceptable salts include the following: acids; including acetic acid, 2,2-dichlorolacetic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and bases; including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The term "treating" or "treatment" as used herein, refers to actions that cause any indicia of success in the prevention or amelioration of an injury, pathology, symptoms or condition, including any objective or subjective parameters such as abatement; remission; diminishing of symptoms or making the injury, pathology, or condition more tolerable to the patient; slowing in the rate of degeneration or decline; making the final point of degeneration less debilitating; or improving a subject's physical or mental well-being.

Thus the term "treatment" or "to treat" is intended to include any action that improves, prevents, reverses, arrests, or inhibits the pathological process of epileptogenesis, as that term is defined and used herein. The treatment or amelioration of symptoms can be based on objective or subjective parameters; including the results of a physical examination, neurological examination, and/or psychiatric evaluations.

Accordingly, the term "treating" or "treatment" includes the administration of the compounds or agents of the present invention to treat, prevent, reverse, arrest, or inhibit the process of epileptogenesis.

The term "therapeutic effect" as used herein, refers to the treatment, inhibition, abatement, reversal, or prevention of epileptogenesis, the effects or symptoms of epileptogenesis, or side effects of epileptogenesis in a subject.

The term "a therapeutically effective amount" or "a therapeutically effective dose" are used interchangeably and, as used herein, mean a sufficient amount or dose of one or more of the compounds or compositions of the invention to produce a therapeutic effect, as defined above, in a subject or patient in need of such; treatment, inhibition, abatement, reversal, or prevention of epileptogenesis, the effects or symptoms of epileptogenesis, or side effects of epileptogenesis. The range of doses required for these different therapeutic effects will differ according to the characteristics of the subject or patient and the precise nature of the condition being treated.

The term "pharmaceutical dosage form" as that term is used herein, shall refer to a form of one or more of the compounds or compositions of this invention along with pharmaceutically acceptable excipients to produce a formulation suitable for administration to a subject. The form may be adapted for administration by any appropriate route including, but not limited to; oral, both immediate and delayed release, intravenous (IV), transdermal, intramuscular, intraventricular or nasal and may comprise; tablets, pills, capsules, semisolids, powders, sustained release formulations, solutions, suspensions, emulsions, syrups, elixirs, aerosols, or any other appropriate compositions.

### Dosage Regimens

The amount of the carbamate compound necessary to treat epileptogenesis is defined as a therapeutically or a pharmaceutically effective amount or dose. In order to accomplish this objective the compounds or compositions of this invention must be used in the correct therapeutically effective amount or dose, as described below

The dosage schedule and amounts effective for this use, i.e., the dosing or dosage regimen, will depend on a variety of factors including the precise nature of the disease or injury, the patient's physical status, weight, age and the like. In calculating the dosage regimen for a patient, the mode of administration is also taken into account.

The range of doses that are expected to be effective in producing an anti-epileptogenic effect in humans in the severe and acute clinical situations that are analogous to the lithium-pilocarpine rat model in Example 2 are determined by comparing known effective doses and blood levels in rats and humans.

In humans, it is known that the pharmacokinetics of one of the compounds of the invention referred to herein as Test Compound (TC), i.e., Formula 7, are linear following single and repeated oral administration in healthy adult men.

### Blood levels in humans;

In toxicology studies in humans, oral administration of Test Compound at various doses for 7 days produced the following C max's and AUC (0-24):
1) At 100 mg. b.i.d. (200 mg in 24 hours or 2.85 mg/kg/day in a 70 kg. human) C max was 3.6-micrograms/mL and AUC was 42.2 micrograms-hour/mL;
2) At 250 mg. b.i.d (500 mg in 24 hours or 7.14 mg/kg/day in a 70 kg human) C max was 8.2 micrograms/mL and AUC was 102.3 micrograms-hour/mL;
3) At 500 mg. b.i.d. (1000 mg in 24 hours or 14.28 mg/kg/day in a 70 kg. human) C max was 17.2-micrograms/mL and AUC was 204.1 micrograms-hour/mL;
4) At 750 mg. b.i.d. (1500 mg in 24 hours or 21.4 mg/kg/day in a 70 kg human) C max was 28.2-micrograms/mL and AUC was 322.7 micrograms-hour/mL.

### Blood levels in rats;

In toxicology studies in rats, oral administration of Test Compound (TC) for 8 days produced the following C max and AUC:
1) At 30 mg/kg/day C max was 9.33 micrograms/ mL and AUC was 97.32 micrograms-hour/mL;
2) At 100 mg/kg/day C max was 20.63 micrograms/ mL and AUC was 230.33 micrograms-hour/mL
3) At 300 mg/kg/day C max was 70.34 micrograms/ mL and AUC was 525.95 micrograms-hour/mL

The doses tested in rats for anti-epileptogenic effects in Example 2 ranged from 30 mg/kg/day to 120 mg/kg/day. The lowest dose tested in this Example, i.e., 30 mg/kg, produced some measurable protective effects while the lowest dose tested in Example 1 was 10 mg/kg/day and produced minimal or no protective effects (See Examples 1 and 2 below). In rats, doses of 30 mg/kg/day of Test Compound (TC) would be expected to produce blood levels of; C max of 9.33 micrograms/mL and an AUC of 97.32 micrograms-hour/mL. In humans, these blood levels would be expected from doses of about 500 mg/day to about 600 mg/day or from about 7.1 to about 8.6 mg/kg/day in a 70 kg human.

However, in Examples 1 and 2 relatively high doses and blood levels were required because of the acute and very severe animal model that was used and the need to produce the dramatic and rapid anti-epileptogenic effects demonstrated. Also, in this severe acute animal model the compound was given after the traumatic event or injury had occurred, i. e., the induction of status epilepticus by administration of Li-Pilocarpine. This type of post injury model is likely to correlate to analogously acute and severe clinical situations in human patients including but not limited to, starting the medication after CNS injury has already occurred. In such situations, it is expected that the dosages needed for an antiepileptogenic effect will be higher than what would likely be needed in less acute or severe circumstances or in chronic situations and especially where the medication is used prophylactically.

In situation where the medication is used prophylactically in a primary prevention or pre-treatment paradigm the required doses and blood levels required to produce clinically important anti-epileptogenic effects would be expected to be somewhat lower than the human equivalent of the 30 mg/kg/day dose used in Examples 2.

As such, the doses expected to be therapeutically effective in clinical practice, in most cases, would be less than that identified in this severe animal model of epileptogenesis. The ED50 for Test Compound for preventing seizures in rats is about 4 mg/kg to about 30 mg/kg (depending on the time and experiment type) so a minimum effective dose of 30 mg/kg in the epileptogenesis rat models is not unexpected. Based on this data, an expected effective antiepileptogenic human dose would be higher than the minimum dose required for anticonvulsant efficacy in humans. In a primary prevention paradigm, where dosing is done well before any insult or pathological process is initiated, the effective doses and blood levels in humans would be expected to be somewhat lower than human equivalent of the 30 mg/kg dose found minimally effective in the lithium-pilocarpine rat model in Examples 1 and 2.

In human patients, in a primary prevention paradigm where the medication would begin prior to any injury or damage to the human nervous system, the lower limits of an anti-epileptogenesis effective dose would be expected to be about 400 mg/day to about 500 mg/day or about 5.7 mg/kg/day to about 7.14 mg/kg/day. In situations where the medication is started after the injury has been sustained the dose range would be expected to be somewhat higher, for example from about 500 mg/day to about 600 mg/day or about 7.14 to about 8.6 mg/kg/day in a 70 kg human.

The compounds and compositions of the invention do not have a theoretical upper end to their clinically effective dose range. Thus, the upper end of the therapeutically effective range would be determined by the maximum amount that could be tolerated by the patient. However, the highest dose tested in rats, i.e., 120 mg/kg, which showed very marked neuroprotective and anti-epileptogenesis effects, would be expected, on the basis of the data above, to have a C max and AUC similar or below those produced in humans at a dose of 750 mg twice a day (1500 mg/day or approximately 21.4 mg/kg/day). This dose was easily tolerated in humans and the maximum tolerable dose would be considerably higher than this for many patients perhaps 2500 to 3000 mg/day or about 35.7 mg/kg/day to about 42.9 mg/kg/day in a 70 kg human.

Thus the pharmaceutical compounds and compositions of the invention may be administered at a dosage of from about 5.7 mg/kg/day to about 43.0 mg/kg/day (400-3000 mg/day in a 70 kg human), preferably from about 6.4 to about 35.7 mg/kg/day (4550-2500 mg/day in a 70 kg human), more preferably from about 7.1 to about 28.6 mg/kg/day (500-2000 mg/day in a 70 kg human), or even more preferably from about 7.8 to about 21.4 mg/kg/day (550-1500 mg/day in a 70 kg human) or most preferably from about 8.6 to about 17.1 mg/kg/day (600-1200 mg/day in a 70 kg human). These dosages, however, may be varied depending the individual characteristics and tolerances of the subject and the on the precise nature of the condition being treated.

Based on this disclosure, a person of ordinary skill in the art will be able, without undue experimentation, having regard to that skill, to determine a therapeutically effective dose or amount of a particular substituted carbamate compound of the invention for treating epilepsy and for producing a clinically significant anti epileptogenic effect. (see, e.g., Lieberman, Pharmaceutical Dosage Forms(Vols. 1-3, 1992); Lloyd, 1999, The art, Science and Technology of Pharmaceutical Compounding; and Pickar, 1999, Dosage Calculations).

A therapeutically effective dose is also one in which any toxic or detrimental side effects of the active agent is outweighed in clinical terms by therapeutically beneficial effects. It is to be further noted that for each particular subject, specific dosage regimens should be evaluated and adjusted over time according to the individual need and professional judgment of the person administering or supervising the administration of the compounds. It is also expected that the compositions of this invention could be initiated at a low or moderate dose and then increased to a fully therapeutically effective dose and blood level over a period of time.

For treatment purposes, the compositions or compounds disclosed herein can be administered to the subject in a single bolus delivery, via continuous delivery over an extended time period, or in a repeated administration protocol (e.g., by an hourly, daily or weekly, repeated administration protocol). The pharmaceutical formulations of the present invention can be administered, for example, one or more times daily, 3 times per week, or weekly. In one embodiment of the present invention, the pharmaceutical formulations of the present invention are orally administered once or twice daily.

In some embodiments, a treatment regimen with the compounds of the present invention can commence, for example, after a subject suffers from a brain damaging injury or other initial insult but before the subject is diagnosed with epilepsy, e.g., before the subject has a first or second seizure. In one embodiment, a subject that is being treated with a compound having epileptogenic potential, e.g., psychotropic drug, or a subject having a disease associated with a risk of developing epilepsy, e.g., autism, can commence a treatment regimen with a carbamate compound of the present invention.

In still other embodiments, a treatment regimen with the compounds of the present invention can commence before any damage or injury to the nervous system has occurred but at a time when such damage or injury can expected or is likely to occur. For example, such a treatment regimen can begin before a subject undergoes a neurosurgical procedure or is likely to suffer other forms or head or brain trauma, e.g., combat, violent sports or racing, recurrent strokes, TIA's etc.

In certain embodiments; the carbamate compounds can be administered daily for a set period of time (week, month, year) after occurrence of the brain damaging injury or initial insult. An attendant physician will know how to determine that the carbamate compound has reached a therapeutically effective level, e.g., clinical exam of a patient, or by measuring drug levels in the blood or cerebro-spinal fluid. One of skill in the art would be able to determine the maximum tolerable dose by means of a physical examination to determine the presence and severity of side effects such as slurred speech, lethargy or impaired coordination.

In this context, a therapeutically effective dosage of the biologically active agent(s) can include repeated doses within a prolonged treatment regimen that will yield clinically significant results to prevent, reverse, arrest, or inhibit the epileptogenesis. Determination of effective dosages in this context is typically based on animal model studies followed up by human clinical trials and is guided by determining effective dosages and administration protocols that significantly reduce the occurrence or severity of targeted exposure symptoms or conditions in the subject. Suitable models in this regard include, for example, murine, rat, porcine, feline, non-human primate, and other accepted animal model subjects known in the art. Alternatively, effective dosages can be determined using in vitro models (e.g., immunologic and histopathologic assays). Using such models, only ordinary calculations and adjustments are typically required to determine an appropriate concentration and dose to administer a therapeutically effective amount of the biologically active agent(s) (e.g., amounts that are intranasally effective, transdermally effective, intravenously effective, or intramuscularly effective to elicit a desired response).

In an exemplary embodiment of the present invention, unit dosage forms of the compounds are prepared for standard administration regimens. In this way, the composition can be subdivided readily into smaller doses at the physician's direction. For example, unit dosages can be made up in packeted powders, vials or ampoules and preferably in capsule or tablet form.

The active compound present in these unit dosage forms of the composition can be present in an amount of, for example, from about 25 mg, to about 800 mg or preferably in unit dosage amounts of about; 50, 100, 200 250, 400, 450, 500, and 600 mg of one or more of the active carbamate compounds of the invention, for single or multiple daily administration, according to the particular need of the patient.

### Carbamate Compounds as Pharmaceuticals:

The present invention provides enantiomeric mixtures and isolated enantiomers of Formula 1 and/or Formula 2 as pharmaceuticals. The carbamate compounds are formulated as pharmaceuticals to treat epileptogenesis.

### Pharmaceutical Compositions

The present invention further comprises pharmaceutical compositions containing one or more compounds of Formula 1 or Formula 2 with a pharmaceutically acceptable carrier.

Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed.

If desired, tablets may be sugar coated or enteric coated by standard techniques. For parenteral use, the carrier will usually comprise sterile water, through other ingredients, for example, for purposes such as aiding solubility or for preservation, may be included. Injectable suspensions may also be prepared, in which case appropriate liquid carriers, suspending agents and the like may be employed.

The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 10 mg to about 1000 mg of one or more compounds of Formula 1 or Formula 2 and preferably unit doses of from about 25 mg to about 800 mg and more preferably in unit doses of about; 50 mg, 100mg, 250 mg, 400mg, 450mg, 500mg and 600mg.

The pharmaceutical compositions may be administered at a dosage of from about 5.7 mg/kg/day to about 43.0 mg/kg/day (400-3000 mg/day in a 70 kg human), preferably from about 6.4 mg/kg/day to about 35.7 mg/kg/day (450-2500 mg/day in a 70 kg human), more preferably from about 7.1 mg/kg/day to about 28.6mg/kg/day (500-2000 mg/day in a 70 kg human), or even more preferably from about 7.9 mg/kg/day to about 21.4 mg/kg/day (550-1500 mg/day in a 70 kg human) or most preferably from about 8.6 to about 17.1 mg/kg/day (600-1200 mg/day in a 70 kg human). The dosages, however, may be varied depending upon the requirements of the patient, the severity of the condition being treated and the compound being employed.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

Preferably these compositions are in unit dosage forms from such as tablets, pills, capsules, powders, granules, sterile parenteral solutions or suspensions, metered aerosol or liquid sprays, drops, ampoules, auto injector devices or suppositories; for oral, parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 25.0 mg to about 800 mg of the active ingredient of the present invention.

The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

One skilled in the art will recognize that, both in vivo and in vitro trials using suitable, known and generally accepted cell and / or animal models are predictive of the ability of a test compound to treat or prevent a given disorder.

One skilled in the art will further recognize that human clinical trails including first-in-human, dose ranging and efficacy trials, in healthy patients and / or those suffering from a given disorder, may be completed according to methods well known in the clinical and medical arts.

In general, the carbamate compounds of the present invention can be administered as pharmaceutical compositions by any method known in the art for administering therapeutic drugs including oral, buccal, topical, systemic (e.g., transdermal, intranasal, or by suppository), or parenteral (e.g., intramuscular, subcutaneous, or intravenous injection.) Administration of the compounds directly to the nervous system can include, for example, administration to intracerebral, intraventricular, intacerebroventricular, intrathecal, intracisternal, intraspinal or peri-spinal routes of administration by delivery via intracranial or intravertebral needles or catheters with or without pump devices.

Compositions can take the form of tablets, pills, capsules, semisolids, powders, sustained release formulations, solutions, suspensions, emulsions, syrups, elixirs, aerosols, or any other appropriate compositions; and comprise at least one compound of this invention in combination with at least one pharmaceutically acceptable excipient. Suitable excipients are well known to persons of ordinary skill in the art, and they, and the methods of formulating the compositions, can be found in such standard references as Alfonso AR: Remington's Pharmaceutical Sciences, 17th ed., Mack Publishing Company, Easton PA, 1985, the disclosure of which is incorporated herein by reference in its entirety and for all purposes. Suitable liquid carriers, especially for injectable solutions, include water, aqueous saline solution, aqueous dextrose solution, and glycols.

The carbamate compounds can be provided as aqueous suspensions. Aqueous suspensions of the invention can contain a carbamate compound in admixture with excipients suitable for the manufacture of aqueous suspensions. Such excipients can include, for example, a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate).

The aqueous suspension can also contain one or more preservatives such as ethyl or n-propyl p-hydroxybenzoate, one or more coloring agents, one or more flavoring agents, and one or more sweetening agents, such as sucrose, aspartame or saccharin. Formulations can be adjusted for osmolarity.

Oil suspensions for use in the present methods can be formulated by suspending a carbamate compound in a vegetable oil, such as arachis oil, olive oil, sesame oil or coconut oil, or in a mineral oil such as liquid paraffin; or a mixture of these. The oil suspensions can contain a thickening agent, such as beeswax, hard paraffin or cetyl alcohol. Sweetening agents can be added to provide a palatable oral preparation, such as glycerol, sorbitol for sucrose. These formulations can be preserved by the addition of an antioxidant such as ascorbic acid. As an example of an injectable oil vehicle, see Minto, J. Pharmacol. Exp. Ther. 281:93-102, 1997. The pharmaceutical formulations of the invention can also be in the form of oil-in-water emulsions. The oily phase can be a vegetable oil or a mineral oil, described above, or a mixture of these.

Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent.

The compound of choice, alone or in combination with other suitable components can be made into aerosol formulations (i.e., they can be "nebulized") to be administered via inhalation. Aerosol formulations can be placed into pressurized acceptable propellants, such as dichlorodifluoromethane, propane, nitrogen, and the like.

Formulations of the present invention suitable for parenteral administration, such as, for example, by intraarticular (in the joints), intravenous, intramuscular, intradermal, intraperitoneal, intraventricular and subcutaneous routes, can include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Among the acceptable vehicles and solvents that can be employed are water and Ringers solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter.

Where the compounds are sufficiently soluble they can be dissolved directly in normal saline with or without the use of suitable organic solvents, such as propylene glycol or polyethylene glycol. Dispersions of the finely divided compounds can be made-up in aqueous starch or sodium carboxymethyl cellulose solution, or in suitable oil, such as arachis oil. These formulations can be sterilized by conventional, well-known sterilization techniques. The formulations can contain pharmaceutical acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like.

The concentration of a carbamate compound in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated according to the known art using those suitable dispersing or wetting agents and suspending agents. The sterile injectable preparation can also be a sterile injectable solution or suspension in a nontoxic parenterally acceptable diluents or solvent, such as a solution of 1,3-butanediol.

These formulations can be presented in unit-dose or multi-dose sealed containers, such as ampoules and vials. Injection solutions and suspensions can be prepared from sterile powders, granules, and tablets of the kind previously described.

A carbamate compound suitable for use in the practice of this invention can be and is preferably administered orally. The amount of a compound of the present invention in the composition can vary widely depending on the type of composition, size of a unit dosage, kind of excipients, and other factors well known to those of ordinary skill in the art. In general, the final composition can comprise, for example, from 1.0% percent by weight (% w) to 90 % w of the carbamate compound, preferably 10 % w to 75 % w, with the remainder being the excipient or excipients.

Pharmaceutical formulations for oral administration can be formulated using pharmaceutically acceptable carriers well known in the art in dosages suitable for oral administration. Such carriers enable the pharmaceutical formulations to be formulated in unit dosage forms as tablets, pills, powder, dragees, capsules, liquids, lozenges, gels, syrups, slurries, suspensions, etc. suitable for ingestion by the patient.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions.

Pharmaceutical preparations for oral use can be obtained through combination of the compounds of the present invention with a solid excipient, optionally grinding a resulting mixture, and processing the mixture of granules, after adding suitable additional compounds, if desired, to obtain tablets or dragee cores. Suitable solid excipients are carbohydrate or protein fillers and include, but are not limited to sugars, including lactose, sucrose, mannitol, or sorbitol; starch from corn, wheat, rice, potato, or other plants; cellulose such as methyl cellulose, hydroxymethyl cellulose, hydroxypropylmethyl-cellulose or sodium carboxymethylcellulose; and gums including arabic and tragacanth; as well as proteins such as gelatin and collagen.

If desired, disintegrating or solubilizing agents can be added, such as the cross-linked polyvinyl pyrrolidone, agar, alginic acid, or a salt thereof, such as sodium alginate. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers.

Lozenge forms can comprise the active ingredient in a flavor, e.g., sucrose, as well as pastilles comprising the active ingredient in an inert base, such as gelatin and glycerin or sucrose and acacia emulsions, gels, and the like containing, in addition to the active ingredient, carriers known in the art.

The compounds of the present invention can also be administered in the form of suppositories for rectal administration of the drug. These formulations can be prepared by mixing the drug with a suitable non-irritating excipient that is solid at ordinary temperatures but liquid at the rectal temperatures and will therefore melt in the rectum to release the drug. Such materials are oocoa butter and polyethylene glycols.

The compounds of the present invention can also be administered by intranasal, intraocular, intravaginal, and intrarectal routes including suppositories, insufflation, powders and aerosol formulations (for examples of steroid inhalants, see Rohatagi, J. Clin. Pharmacol. 35:1187-1193,1995; Tjwa, Ann. Allergy Asthma Immunol. 75:107-111, 1995).

The compounds of the present invention can be delivered transdermally, by a topical route, formulated as applicator sticks, solutions, suspensions, emulsions, gels, creams, ointments, pastes, jellies, paints, powders, and aerosols.

Encapsulating materials can also be employed with the compounds of the present invention and the term "composition" can include the active ingredient in combination with an encapsulating material as a formulation, with or without other carriers. For example, the compounds of the present invention can also be delivered as microspheres for slow release in the body. In one embodiment, microspheres can be administered via intradermal injection of drug (e.g., mifepristone)-containing microspheres, which slowly release subcutaneously (see Rao, J. Biomater Sci. Polym. Ed. 7:623-645, 1995; as biodegradable and injectable gel formulations (see, e.g., Gao, Pharm. Res. 12:857-863, 1995); or, as microspheres for oral administration (see, e.g., Eyles, J. Pharm. Pharmacol. 49:669-674, 1997). Both transdermal and intradermal routes afford constant delivery for weeks or months. Cachets can also be used in the delivery of the compounds of the present invention.

The compositions of this invention can be administered in a variety of oral dosage form adapted for slow or controlled release. For example, the composition can be placed in an insoluble capsule with a hole at one end and a fluid absorbing distensible composition within the capsule opposite the perforated end. After administration, the fluid absorbing composition absorbs water from the patient's GI tract and swells and forces the active drug out through the perforation at a known and controllable rate. Many other delayed release or controlled release dosage forms known in the art can also be used in conjunction with the methods and compositions of this invention

In another embodiment, the compounds of the present invention can be delivered by the use of liposomes which fuse with the cellular membrane or are endocytosed, i.e., by employing ligands attached to the liposome that bind to surface membrane protein receptors of the cell resulting in endocytosis. By using liposomes, particularly where the liposome surface carries ligands specific for target cells, or are otherwise preferentially directed to a specific organ, one can focus the delivery of the carbamate compound into target cells in vivo. (See, e.g., Al-Muhammed, J. Microencapsul. 13:293-306, 1996; Chonn, Curr. Opin. Biotechnol. 6:698-708, 1995; Ostro, Am. J. Hosp. Pharm. 46:1576-1587, 1989).

The pharmaceutical formulations of the invention can be provided as a salt and can be formed with many acids, including but not limited to hydrochloric, sulfuric, acetic, lactic, tartaric, malic, succinic, etc. Salts tend to be more soluble in aqueous or other protonic solvents that are the corresponding free base forms.

In other cases, the preferred preparation can be a lyophilized powder which can contain, for example, any or all of the following: 1 mM-50 mM histidine, 0.1 %-2% sucrose, 2%-7% mannitol, at a pH range of 4.5 to 5.5, that is combined with buffer prior to use.

Pharmaceutically acceptable salts and esters refer to salts and esters that are pharmaceutically acceptable and have the desired pharmacological properties. Such salts include salts that may be formed where acidic protons present in the compounds are capable of reacting with inorganic or organic bases. Suitable inorganic salts include those formed with the alkali metals, e.g. sodium and potassium, magnesium, calcium, and aluminum. Suitable organic salts include those formed with organic bases such as the amine bases, e.g. ethanolamine, diethanolamine, triethanolamine, tromethamine, N methylglucamine, and the like.

Pharmaceutically acceptable salts can also include acid addition salts formed from the reaction of amine moieties in the parent compound with inorganic acids (e.g. hydrochloric and hydrobromic acids) and organic acids (e.g. acetic acid, citric acid, maleic acid, and the alkane- and arene-sulfonic acids such as methanesulfonic acid and benzenesulfonic acid). Pharmaceutically acceptable esters include esters formed from carboxy, sulfonyloxy, and phosphonoxy groups present in the compounds. When there are two acidic groups present, a pharmaceutically acceptable salt or ester may be a mono-acid-mono-salt or ester or a di-salt or ester; and similarly where there are more than two acidic groups present, some or all of such groups can be salified or esterified.

Compounds named in this invention can be present in unsalified or unesterified form, or in salified and/or esterified form, and the naming of such compounds is intended to include both the original (unsalified and unesterified) compound and its pharmaceutically acceptable salts and esters. The present invention includes pharmaceutically acceptable salt and ester forms of Formula 1 and Formula 2. More than one crystal form of an enantiomer of Formula 1 or Formula 2 can exist and as such are also included in the present invention.

A pharmaceutical composition of the invention can optionally contain, in addition to a carbamate compound, at least one other therapeutic agent useful in the treatment of a disease or condition associated with epilepsy or epileptogenesis or an analogous seizure related disorder

Methods of formulating pharmaceutical compositions have been described in numerous publications such as Pharmaceutical Dosage Forms: Tablets. Second Edition. Revised and Expanded. Volumes 1-3, edited by Lieberman et al; Pharmaceutical Dosage Forms: Parenteral Medications. Volumes 1-2, edited by Avis et al; and Pharmaceutical Dosage Forms: Disperse Systems. Volumes 1-2, edited by Lieberman et al; published by Marcel Dekker, Inc, the disclosure of which are herein incorporated by reference in their entireties and for all purposes.

The pharmaceutical compositions are generally formulated as sterile, substantially isotonic and in full compliance with all Good Manufacturing Practice (GMP) regulations of the U.S. Food and Drug Administration.

The following Examples are set forth to aid in the understanding of the invention, and are not intended and should not be construed to limit in any way the invention set forth in the claims which follow thereafter.

### EXAMPLES

The activity of an isolated S-enantiomer of Formula 1 (e.g., Formula 7), herein referred to as the "Test Compound" or "TC" or "test compound" was evaluated in the following experiments to determine the efficacy of the compound for neuroprotection and in the treatment of epileptogenesis in the model of temporal lobe epilepsy induced by lithium and pilocarpine in the rat.

### Example 1

### The lithium-pilocarpine model of temporal lobe epilepsy

The model induced in rats by pilocarpine associated with lithium (Li-Pilo) reproduces most of the clinical and neurophysiological features of human temporal lobe epilepsy (Turski et al., 1989, Synapse 3:154-171; Cavalheiro, 1995, Ital J Neurol Sci 16:33-37). In adult rats, the systemic administration of pilocarpine leads to status epilepticus (SE). The lethality rate reaches 30-50% during the first days. In the surviving animals, neuronal damage predominates within the hippocampal formation, the piriform and entorhinal cortices, thalamus, amygdaloid complex, neocortex and substantia nigra. This acute seizure period is followed by a "silent" seizure-free phase lasting for a mean duration of 14-25 days after which all animals exhibit spontaneous recurrent convulsive seizures at the usual frequency of 2 to 5 per week (Turski et al., 1989, Synapse 3:154-171; Cavalheiro, 1995, Ital J Neurol Sci 16:33-37; Dube et al., 2001, Exp Neurol 167:227-241).

### Lithium-pilocarpine and treatments with the test compound

Male Wistar rats weighing 225-250 g, provided by Janvier Breeding Center (Le Genest-St-Iste, France) were housed under controlled standard conditions (light/dark cycle, 7.00 a.m.-7.00 p.m. lights on), with food and water available ad libitum. All animal experimentation was performed in accordance with the rules of the European Communities Council Directive of November 24,1986 (86/609/EEC), and the French Department of Agriculture (License N° 67-97). For electrode implantation, rats were anesthetized by an i.p. injection of 2.5 mg/kg diazepam (DZP, Valium, Roche, France) and 1 mg/kg ketamine chlorhydrate (Imalgene 1000, Rhone Merrieux, France). Four single-contact recording electrodes were placed on the skull, over the parietal cortex, two on each side.

### Status epilepticus induction:

### Treatment with the test compound and occurrence of spontaneous recurrent seizures (SRS)

All rats received lithium chloride (3 meq/kg, i.p., Sigma, St Louis, Mo, U.S.A.); about 20 h later, animals were placed into plexiglas boxes, in order to record baseline cortical EEG. Methylscopolamine bromide (1 mg/kg, s.c., Sigma) was administered to limit the peripheral effects of the convulsant. SE was induced by injecting pilocarpine hydrochloride (25 mg/kg, s.c., Sigma) 30 min after methyl-scopolamine. The bilateral EEG cortical activity was recorded during the whole duration of SE and behavioral changes were noted.

The effects of increasing doses of the Test Compound were studied on 3 groups of rats. The animals of the first group received 10 mg/kg of the test compound, i.p., 1 h after the onset of SE (pilo-TC10) while the animals of groups 2 and 3 received 30 and 60 mg/kg of the Test Compound (pilo-TC30 and pilo-TC60), respectively.

Another group was injected with 2 mg/kg diazepam (DZP, i.m.) at 1 h after the onset of SE which are our standard treatment to improve animals survival after SE (pilo-DZP). The control group received saline instead of pilocarpine and the Test Compound (saline-saline). The pilo-Test Compound rats surviving SE where then injected about 10 h after the first test compound injection with a second i.p. injection of the same dose of the test compound and were maintained under a twice daily treatment with the test compound for 6 additional days. Pilo-DZP received a second injection of 1 mg/kg DZP on the day of SE at about 10 h after the first one. Thereafter, Pilo-DZP and saline-saline rats received twice daily an equivalent volume of saline.

The effects of the test compound on the EEG and on the latency to occurrence of SRS were investigated by daily video recording of the animals for 10 h per day and the recording of the electrographic activity twice a week for 8 h.

### Quantification of cell densities

Quantification of cell densities was performed at 6 days after SE on 8 pilo-DZP, 8 pilo-TC10, 7 pilo-TC30, 7 pilo-TC60, and 6 saline-saline rats. At 14 days after SE, animals were deeply anesthetized with 1.8 g/kg pentobarbital (Dolethal®, Vetoquinol, Lure, France. Brains were then removed and frozen. Serial 20 µm slices were cut in a cryostat, air-dried during several days before thionine staining.
■ Quantification of cell densities was performed with a 10 x 10 boxes 1 cm² microscopic grid on coronal sections according to the stereotaxic coordinates of the rat brain atlas. (See Paxinos G, Watson C (1986) The Rat Brain in Stereotaxic Coordinates, 2nd ed. Academic Press, San Diego)
■ Cell counts were performed twice in a blind manner and were the average of at least 3 values from 2 adjacent sections in each individual animal. Counts involved only cells larger than 10 µm, smaller ones being considered as glial cells.

### Timm staining

At 2 months after the onset of spontaneous recurrent seizures, mossy fiber sprouting was examined on rats in the chronic period exposed to the test compound or DZP and in 3 saline-saline rats. Animals were deeply anaesthetized and perfused transcardially with saline followed by 100 ml of 1.15% (w/v) Na₂S in 0.1 M phosphate buffer, and 100 ml of 4% (v/v) formaldehyde in 0.1 M phosphate buffer. Brains were removed from skull, post-fixed in 4% formaldehyde during 3-5 h and 40 µm sections were cut on a sliding vibratome and mounted on gelatin-coated slides.

The following day, sections were developed in the dark in a 26°C solution of 50% (w/v) arabic gum (160 ml), sodium citrate buffer (30 ml), 5.7% (w/v) hydroquinone (80 ml) and 10% (w/v) silver nitrate (2.5 ml) during 40-45 min. The sections were then rinsed with tap water at 40°C during at least 45 min, rinsed rapidly with distilled water and allowed to dry. They were dehydrated in ethanol and coverslipped.

Mossy fiber sprouting was evaluated according to criteria previously described in dorsal hippocampus (Cavazos et al., 1991, J Neurosci 11:2795-2803.), which are follows: 0 - no granules between the tips and crest of the DG; 1 - sparse granules in the supragranular region in a patchy distribution between the tips and crest of DG; 2 - more numerous granules in a continuous distribution between the tips and crest of DG; 3 - prominent granules in a continuous pattern between tips and crest, with occasional patches of confluent granules between tips and crest; 4 - prominent granules that form a confluent dense laminar band between tips and crest and 5 - confluent dense laminar band of granules that extends into the inner molecular layer.

### Data analysis

For the comparison of the characteristics of SE in pilo-saline and pilo-test compound animals, a non-paired Student's t-test was used. The comparison between the number of rats seizing in both groups was performed by means of a Chi square test. For neuronal damage, statistical analysis between groups was performed using ANOVA followed by a Fisher's test for multiple comparisons using the Statview software (Fisher RA, 1946a, Statistical Methods for Research Workers (10th edition) Oliver & Boyd, Edinburgh; Fisher RA, 1946b, The Design of Experiments (4th edition) Oliver & Boyd, Edinburgh)

### Behavioral and EEG characteristics of lithium-pilocarpine status epilepticus

A total number of Sprague-Dawley rats weighing 250-330 g were subjected to Li-pilo induced SE. The behavioral characteristics of SE were identical in both pilo-saline and pilo-test compound groups. Within 5 min after pilocarpine injection, rats developed diarrhea, piloerection and other signs of cholinergic stimulation. During the following 15-20 min, rats exhibited head bobbing, scratching, chewing and exploratory behavior. Recurrent seizures started around 15-20 min after pilocarpine administration. These seizures which associated episodes of head and bilateral forelimb myoclonus with rearing and falling progressed to SE at about 35-40 min after pilocarpine, as previously described (Turski et al., 1983, Behav Brain Res 9:315-335.).

### EEG patterns during SE

During the first hour of SE, in the absence of pharmacological treatment, the amplitude of the EEG progressively increased while the frequency decreased. Within 5 min after the injection of pilocarpine, the normal background EEG activity was replaced with low voltage fast activity in the cortex while theta rhythm (5-7 Hz) appeared in the hippocampus. By 15-20 min, high voltage fast activity superposed over the hippocampal theta rhythm and isolated high voltage spikes were recorded only in the hippocampus while the activity of the cortex did not substantially change.

By 35-40 min after pilocarpine injection, animals developed typical electrographic seizures with high voltage fast activity present in both the hippocampus and cortex which first occurred as bursts of activity preceding seizures and were followed by continuous trains of high voltage spikes and polyspikes lasting until the administration of DZP or the test compound. At about 3-4 h of SE, the hippocampal EEG was characterized by periodic electrographic discharges (PEDs, about one/sec) in the pilo-DZP and in the pilo-10 group in both hippocampus and cortex. The amplitude of EEG background activity was low in the pilo-TC60 animals. By 6-7 h of SE, spiking activity was still present in the cortex and the hippocampus in the DZP-and TC10-treated rats while the amplitude of the EEG decreased and came back to baseline levels in the hippocampus of TC30 rats and in both structures of TC60 treated rats. There was no difference between TC10, TC30, and TC60 groups. By 9 h of SE, isolated spikes were still recorded in the hippocampus of test compound-treated rats and occasionally in the cortex. In both structures, the background activity was of very low amplitude at that time.

### Mortality induced by SE

During the first 46 h after SE, the degree of mortality was similar in pilo-DZP rats (23%, 5/22), pilo-TC10 rats (26%, 6/23), and pilo-TC30 rats (20%, 5/25), The mortality rate was largely reduced in pilo-TC60 rats in which it only reached 4%(1/23). The difference was statistically significant (p <0.01).

### EEG characteristics of the silent phase and occurrence of spontaneous recurrent seizures

The EEG patterns during the silent period were similar in pilo-DZP and pilo-TC10, 30 or 60 rats. At 24 and 48 h days after SE, the baseline EEG was still characterized by the occurrence of PEDs on which large waves or spikes could be superimposed. Between 1 and 8 h after injection of the test compound or vehicle injection, there was no change in the pilo-DZP or pilo-TC10 groups. In TC30 and TC60 rats, the frequency and amplitude of PEDs decreased as soon as 10 min after injection and were replaced by spikes of large amplitude in the TC30 group and of low amplitude in the TC60 group. By 4 h after injection the EEG had returned to baseline levels in the two latter groups. By 6 days after SE, the EEG was still of lower amplitude than before pilocarpine injection and in most groups spikes could still be recorded, occasionally in the pilo-DZP, -TC10 and -TC30 rats. In pilo-TC60 rats, the frequency of large amplitude spikes was higher than in all other groups.

After the test compound or vehicle-injection, EEG recording was not affected by the injection in the pilo-DZP and pilo-TC10 groups. In pilo-TC30 rats, the injection induced the occurrence of slow waves on the EEG of both hippocampus and cortex and a decreased frequency of spikes in the pilo-TC60 rats.

All the rats exposed to DZP, TC10 and TC30 and studied until the chronic phase developed SRS with a similar latency. The latency was 18.2 ± 6.9 days (n = 9) in pilo-DZP rats, 15.4 ±5.1 days (n = 7) in pilo-TC10 rats, 18.9 ± 9.0 days (n = 10) in pilo-TC30 rats. In the group of rats subjected to TC60, a subgroup of rats became epileptic with a latency similar to that of the other groups, i.e. 17.6± 8.7 days (n= 7) while another group of rats became epileptic with a much longer delay ranging from 109 to 191 days post-SE (149.8 ± 36.0 days, n = 4) and one rat did not become epileptic in a delay of 9 months post-SE. The difference in the latency to SRS between pilo-DZP, pilo-TC10, pilo-TC30 and the first subgroup of pilo-TPM60 rats was not statistically significant. None of the saline-saline rats (n =5) developed SRS.

To calculate the frequency of SRS in pilocarpine-exposed rats, the seizure severity and distinguished stage III (clonic seizures of facial muscles and anterior limbs) and stage IV-V seizures (rearing and falling) was measured. The frequency of stage III SRS per week in pilo-DZP and pilo-test compound rats was variable amongst the groups. It was low, constant in the pilo-DZP and pilo-TC60 (with early SRS onset) groups during the first 3 weeks and had disappeared during the 4th week in the pilo-DZP group. The frequency of stage III SRS was higher in the pilo-TC10 group where it was significantly increased over pilo-DZP values during weeks 3 and 4. The frequency of more severe stage IV-V SRS was highest during the first week in most groups, except pilo-TC30 and TC60 with late seizure onset where the SRS frequency was constant over the whole 4 weeks in TC30 group and over the first two weeks in the pilo-TC60 group with late SRS onset in which no stage IV-V seizures where no seizures recorded after the second week. The frequency of stage IV-V SRS was significantly reduced in the TC10, TC30 and TC60 (with early SRS onset) groups (2.3-6,1 SRS per week) compared to the pilo-DZP group (11.3 SRS per week) during the first week. During weeks 2-4v the frequency of stage IV-V SRS was reduced in all groups compared to the first week reaching values of 2-6 seizures per week, except in the pilo-TC60 group with early SRS onset where the frequency of seizures was significantly reduced to 0.6-0.9 seizure per week compared to the pilo-DZP group in which the frequency of SRS ranged from 3.3 to 5.8.

### Cell densities in hippocampus, thalamus and cortex

In pilo-DZP rats compared to saline-saline rats, the number of cells was massively decreased in the CA1 region of the hippocampus (70% cell loss in the pyramidal cell layer) while the CAS region was less extensively damaged (54% cell loss in CA3a and 31% in CA3b). In the dentate gyrus, the pilo-DZP rats experienced extensive cell loss in the hilus (73%) while the granule cell layer did not show visible damage. Similar damage was observed in the ventral hippocampus but cell counts were not performed in this region. Extensive damage was also recorded in the lateral thalamic nucleus (91 % cell loss) while the mediodorsal thalamic nucleus was more moderately damaged (56%). In the piriform cortex, cell loss was total in layers III-IV which was no longer visible and reached 53% in layer II in pilo-DZP rats. In the dorsal entorhinal cortex, layers II and III-IV underwent slight damage (9 and 15%, respectively). Layer II of the ventral entorhinal cortex was totally preserved while layers III-IV underwent a 44% cell loss.

In the hippocampus of pilo-test compound animals, cell loss was reduced compared to pilo-DZP rats in the CA1 pyramidal layer in which the cell loss reached 75% in pilo-DZP and 35 and 16% in the pilo-TC30 or pilo-TC60 animals, respectively. This difference was statistically significant at the two test compound doses. In the CAS pyramidal layer, the test compound did not afford any protection in the CA3a area while the 60 mg/kg of the test compound dose was significantly neuroprotective in CA3b. In the dentate gyrus, the cell loss in the hilus was similar in pilo-test compound (69-72%) and pilo-DZP animals (73%). In the two thalamic nuclei, the 60 mg/kg dose was also protective in reducing neuronal damage by 65 and 42% in the lateral and mediodorsal nucleus, respectively. In the cerebral cortex, the treatment with the test compound afforded neuronal protection compared to DZP only at the highest dose, 60 mg/kg. At the two lowest doses, 10 and 30 mg/kg, the total loss of cells and tissue disorganization observed in layers III-IV of the piriform cortex was identical in pilo-DZP rats and pilo-test compound rats and did not allow any counting in any of the groups. In layers II and III-IV of the piriform cortex, the TC60 treatment reduced neuronal damage recorded in the pilo-DZP rats by 41 and 44%, respectively. In the ventral entorhinal cortex, neuroprotection was induced by TC60 administration in layers III-IV and reached 31% compared to pilo-DZP rats. In the entorhinal cortex, there was a slight worsening of cell loss in pilo-TC10 rats compared with pilo-DZP rats in layers III-IV of the dorsal entorhinal cortex (28% more damage) and layers III-IV of the ventral entohinal cortex (35% more damage). At the other doses of the test compound, cell loss in the entorhinal cortex was similar to the one recorded in pilo-DZP rats.

### Mossy fiber sprouting in hippocampus

All rats exhibiting SRS in pilo-DZP and pilo-TPM groups showed similar intensity of Timm staining in the inner molecular layer of the dentate gyrus (scores 2-4). Timm staining was present both on the upper and lower blades of the dentate gyrus. The mean value of the Timm score in the upper blade reached 2.8 ± 0.8 in pilo-DZP rats (n = 9), 1.5 ±0.6 in pilo-TC10 rats (n = 7), 2.6 ± 1.0 in pilo-TC30 rats (n = 10), and 1.5 ± 0.7 in the whole group of pilo-TC60 rats (n = 11). When the pilo-test compound at 60mg/kg.group was subdivided according to the latency to SRS, the subgroup with early SRS occurrence showed a Timm score of 1.8 ± 0.6 (n = 6) and the subgroup of rats with late occurrence or absence of SRS had a Timm score of 1.2 ± 0.6 (n = 5). The values recorded in the pilo-DZP rats were statistically significantly different from the values in the pilo-TC10 (p = 0.032) and the pilo-TC60 subgroup with late or no seizures (p = 0.016).

### Discussion and conclusions

The results of the present study show that a 7-day treatment with the test compound starting at 1 h after the onset of SE is able to protect some brain areas from neuronal damage, e.g., in the pyramidal cell layer of the CA1 and CA3b area, the mediodorsal thalamus, layers II and II MV of the piriform cortex and layers III-IV of the ventral entorhinal cortex, but only at the highest dose the test compound, i.e. 60 mg/kg. The latter dose of the test compound is also able to delay the occurrence of SRS, at least in a subgroup of animals that became epileptic with a mean delay that was about 9-fold longer than in the other groups of animals and one animal did not become epileptic in a delay of 9 months after SE.

These results show that one compound with anti-ictal properties, which are the classical properties of most antiepileptic-marketed drugs, is also able to delay epileptogenesis, i.e. to be antiepileptogenic. The data of the present study show also that the test compound treatment, whatever the dose used, decreases the severity of the epilepsy since it decreases the number of stage IV-V seizures, mainly during the first week of occurrence and during the whole period of 4-weeks observation with the test compound at 60mg/kg. treatment. Moreover, in the TC10 group, there is a shift to an increase in the occurrence of less severe stage III seizures that are more numerous than in the pilo-DZP group.

### EXAMPLE 2

The aim of this extended portion of the study was to pursue the study reported in Example 1 above on the potential neuroprotective and antiepileptogenic properties of the same Test Compound (TC) in the lithium-pilocarpine (Li-Pilo) model of temporal lobe epilepsy. In the first study it was shown that TC was able to protect areas CA1 and CA3 of the hippocampus, piriform and ventral entorhinal cortex from neuronal damage induced by Li-Pilo status epilepticus (SE). Most of these neuroprotective properties occurred at the highest dose studied, 60 mg/kg and the treatment was able to delay the occurrence of spontaneous seizures in 36% (4 out of 11) of the rats. In the present example, the consequences of treatment by higher doses of TC on neuronal damage and epileptogenesis is studied

### The lithium-pilocarpine model of temporal lobe epilepsy

The model of epilepsy induced in rats by pilocarpine associated with lithium (Li-Pilo) reproduces most of the clinical and neurophysiological features of human temporal lobe epilepsy (See, Turski L, Ikonomidou C, Turski WA, Bortolotto ZA, Cavalheiro EA (1989) Review: Cholinergic mechanisms and epileptogenesis. The seizures induced by pilocarpine: a novel experimental model of intractable epilepsy, Synapse 3:154-171; Cavalheiro EA (1995) The pilocarpine model of epilepsy. Ital J Neurol Sci 16:33-37).

In adult rats, the systemic administration of pilocarpine leads to SE which may last for up to 24 h. The lethality rate reaches 30-50% during the first days. In the surviving animals, neuronal damage predominates within the hippocampal formation, the piriform and entorhinal cortices, thalamus, amygdaloid complex, neocortex and substantia nigra. This acute seizure period is followed by a "silent" seizure-free phase lasting for a mean duration of 14-25 days after which all animals exhibit spontaneous recurrent convulsive seizures at the usual frequency of 2 to 5 per week (See, Turski L, Ikonomidou C, Turski WA, Bortolotto ZA, Cavalheiro EA (1989) Review: Cholinergic mechanisms and epileptogenesis, The seizures induced by pilocarpine: a novel experimental model of intractable epilepsy Synapse 3:154-171; Cavalheiro EA (1995) The pilocarpine model of epilepsy. Ital J Neurol Sci 16:33-37; Dubé C, Boyet S, Marescaux C, Nehlig A (2001) Relationship between neuronal loss and interictal glucose metabolism during the chronic phase of the lithium-pilocarpine model of epilepsy in the immature and adult rat. Exp Neurol 167:227-241)).

The current antiepileptic drugs (AED's) do not prevent epileptogenesis and are only transiently efficient on recurrent seizures.

In our previous study, we studied the potential neuroprotective and antiepileptogenic effects of increasing doses of Test Compound (TC) given in monotherapy and compared to our standard diazepam (DZP) treatment mostly given to prevent high mortality. These data show that a 7-day treatment with 10, 30 or 60 mg/kg TC starting at 1 h after the onset of SE is able to protect some brain areas from neuronal damage. This effect is statistically significant in the pyramidal cell layer of the CA1 and CA3b area, the mediodorsal thalamus, layers II and III-IV of the piriform cortex and layers III-IV of the ventral entorhinal cortex, but only at the highest dose of TC, i.e. 60 mg/kg. Moreover, it appears that the latter dose of TC is also the only one that is able to delay the occurrence of SRS, at least in a subgroup of animals that became epileptic with a mean delay that was about 9-fold longer than in the other groups of animals and one animal did not become epileptic in a delay of 9 months after SE.

In the present study, the effects of different doses of Test Compound (TC), i.e. 30, 60, 90 and 120 mg/kg (TC30, TC60, TC90 and TC120) were tested using the same design as in the previous study. The treatment was started one hour after the onset of SE and the animals were treated with a second injection of the same dose of the drug. This early treatment of SE was followed by a 6 days TC treatment. This report concerns the effects of the four different doses of TC on neuronal damage assessed in hippocampus, parahippocampal cortices, thalamus and amygdala at 14 days after SE and on the latency to and frequency of spontaneous epileptic seizures.

### Methods

### Animals

Adult male Sprague-Dawley rats provided by Janvier Breeding Center (Le Genest-St-Isle, France) were housed under controlled, uncrowded standard conditions at 20-22°C (light/dark cycle, 7.00 a.m.-7.00 p.m. lights on), with food and water available ad libitum. All animal experimentation was performed in accordance with the rules of the European Communities Council Directive of November 24, 1986 (86/609/EEC), and the French Department of Agriculture (License N° 67-97).

### Status epilepticus induction, Test Compound (TC) treatment and occurrence of SRS

All rats received lithium chloride (3 meq/kg, i.p., Sigma, St Louis, Mo, U.S.A.) and about 20 h later, all animals received also methylscopolamine bromide (1 mg/kg, s.c., Sigma) that was administered to limit the peripheral effects of the convulsant. SE was induced by injecting pilocarpine hydrochloride (25 mg/kg, s.c., Sigma) 30 min after methylscopolamine. The effects of increasing doses of TC were studied in 5 groups of rats. The animals received either 2.5 mg/kg DZP, i.m., or 30, 60, 90 or 120 mg/kg TC (TC30 , TC60 , TC90 , TC120), i.p., at 1 h after the onset of SE. The control group received vehicle instead of pilocarpine and TC. The rats surviving SE were then injected about 10 h after the first TC injection with a second i.p. injection of 1.25 mg/kg DZP for the DZP group or of the same dose of TC as in the morning and were maintained under a twice daily TC treatment (s.c.) for 6 additional days while DZP rats received a vehicle injection.

The effects of DZP and the 4 doses of TC on epileptogenesis were investigated by daily video recording of the animals for 10 h per day. Video recording was performed for 4 weeks during which the occurrence of the first seizure was noted as well as the total number of seizures over the whole period. Animals were then taken off the video recording system and kept for 4 additional weeks in our animal facilities before they were sacrificed after a total period of 8 weeks of epilepsy. The rats that did not exhibit seizures were sacrificed after 5 months of video recording.

### Quantification of cell densities

Quantification of cell densities was performed at two times after SE: a first group was studied 14 days after SE and was composed by 7 DZP, 8 TC30 , 11 TC60 , 10 TC90 , 8 TC120 and 8 control rats not subjected to SE. A second group used for the study of the latency to SRS was sacrificed either 8 weeks after the first SRS or at 5 months when no SRS could be seen in that delay and was composed of 14 DZP, 8 TC30 , 10 TC60 , 11 TC90 , 9 TC120 rats. At the moment, neuronal counting is still in progress in the second group of animals studied for epileptogenesis and long-term counting and the data concerning that part of the study will not be included in the present report.

For neuronal counting, animals were deeply anesthetized with 1.8 g/kg pentobarbital (Dolethal®, Vétoquinol, Lure, France). Brains were then removed and frozen. Serial 20 µm slices were cut in a cryostat, air-dried during several days before thionine staining. Quantification of cell densities was performed with a 10 x 10 boxes 1 cm² microscopic grid on coronal sections according to the stereotaxic coordinates of the rat brain atlas (Paxinos G, Watson C (1986) The Rat Brain in Stereotaxic Coordinates, 2nd ed. Academic Press, San Diego). The grid of counting was placed on a well defined area of the cerebral structure of interest and counting was carried out with a microscopic enlargement of 200- or 400-fold defined for each single cerebral structure. Cell counts were performed twice on each side of three adjacent sections for each region by a single observer unaware of the animal's treatment. The number of cells obtained in the 12 counted fields in each cerebral structure was averaged. This procedure was used to minimize the potential errors that could result from double counting leading to overestimation of cell numbers. Neurons touching the inferior and right edges of the grid were not counted. Counts involved only neurons with cell bodies larger than 10 µm. Cells with small cell bodies were considered as glial cells and were not counted.

### Data analysis

For neuronal damage and epileptogenesis, statistical analysis between groups was performed by means of a one-way analysis of variance followed by a post-hoc Dunnett or Fisher test using the Statistica software.

### Results

### Behavioral characteristics of lithium-pilocarpine status epilepticus

A total number of 143 Sprague-Dawley rats weighing 250-330 g were subjected to lithium-pilocarpine -(Li-pilo)-induced SE. In this number 10 did not develop SE while 133 rats developed a full characteristic Li-pilo SE. The behavioral characteristics of SE were identical in both li-pilo-DZP and li-pilo-TC groups. Within 5 min after pilocarpine injection, rats developed diarrhea, piloerection and other signs of cholinergic stimulation. During the following 15-20 min, rats exhibited head bobbing, scratching, chewing and exploratory behavior. Recurrent seizures started around 15-20 min after pilocarpine administration. These seizures which associated episodes of head and bilateral forelimb myoclonus with rearing and falling progressed to SE at about 35-40 min after pilocarpine, as previously described (Turski L, Ikonomidou C, Turski WA, Bortolotto ZA, Cavalheiro EA (1989) Review: Cholinergic mechanisms and epileptogenesis. The seizures induced by pilocarpine: a novel experimental model of intractable epilepsy. Synapse 3:154-171; Dubé C, Boyet S, Marescaux C, Nehlig A (2001) Relationship between neuronal loss and interictal glucose metabolism during the chronic phase of the lithium-pilocarpine model of epilepsy in the immature and adult rat. Exp Neurol 167:227-241; André V, Rigoulot MA, Koning E, Ferrandon A, Nehlig A (2003) Long-term pregabalin treatment protects basal cortices and delays the occurrence of spontaneous seizures in the lithium-pilocarpine model in the rat. Epilepsia 44:893-903). The control group not subjected to SE and receiving lithium and saline was composed of 20 rats.

In the group of 57 animals devoted to cell counting at 14 days after SE, a total number of 13 rats died over the first 48 h after SE. The degree of mortality varied with the treatment: 36% (4/11) of DZP rats, 33% (4/12) of TC30 rats, 8% (1/12) of TC60 rats, 0%(0/10) of TC90 rats and 33% (4/12) of TC120 rats died. In the DZP group, the 4 rats died in the first 24 h after SE. In the group of TC30 rats, one rat died on the day of SE, one rat was dead by 24 h after SE and two rats by 48 h. In the group of TC60 rats, one rat died at 48 h after SE. In the group of TC120 rats, two rats were dead by 24 h and two by 48 h after SE.

In the group of 55 animals devoted to the study of the latency to SRS and late cell counting, the degree of mortality over the first 48 h after SE was the following: 7% (1/14) of DZP rats, 27% (3/11) of TC30 rats, 0% (0/10) of TC60 rats, 0% (0/11) of TC90 rats and 0% (0/9) of TC120 rats died. In the group of DZP rats, one rat died during the first 24 h after SE. In the group of TC30 , two rats were dead by 24 h and one by 48 h after SE.

### Cell densities in hippocampus and cortex in the early phase (14 days after SE)

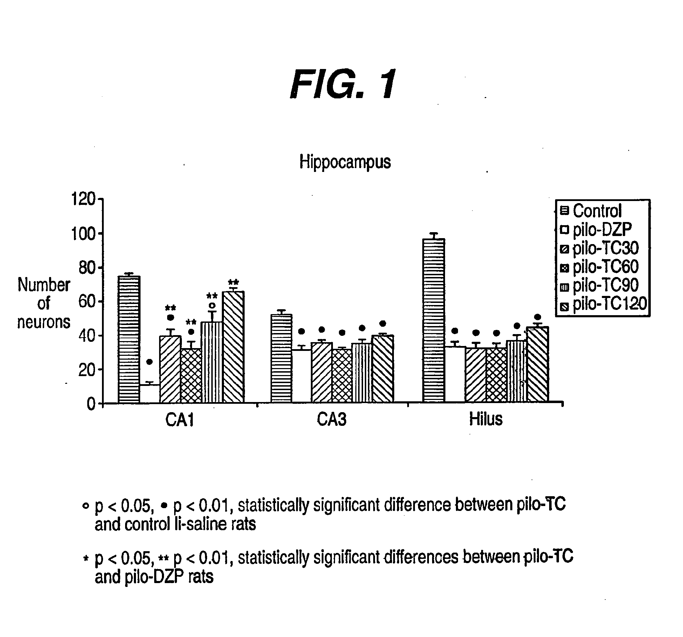

In DZP rats compared to control rats, the number of neurons was massively decreased in the CA1 region of the hippocampus (85% drop out in the pyramidal cell layer) while the CA3 region was less extensively damaged (40% loss) (Table 1 and Figure 1). In the dentate gyrus, DZP rats experienced extensive neuronal loss in the hilus (65%) while the granule cell layer did not show overt damage. The same distribution of damage was observed in the ventral hippocampus but cell counts were not performed in this region.

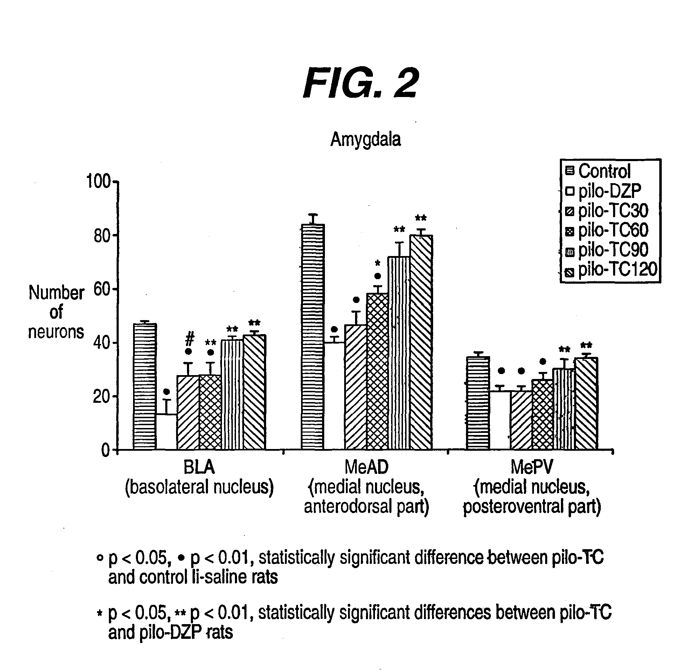

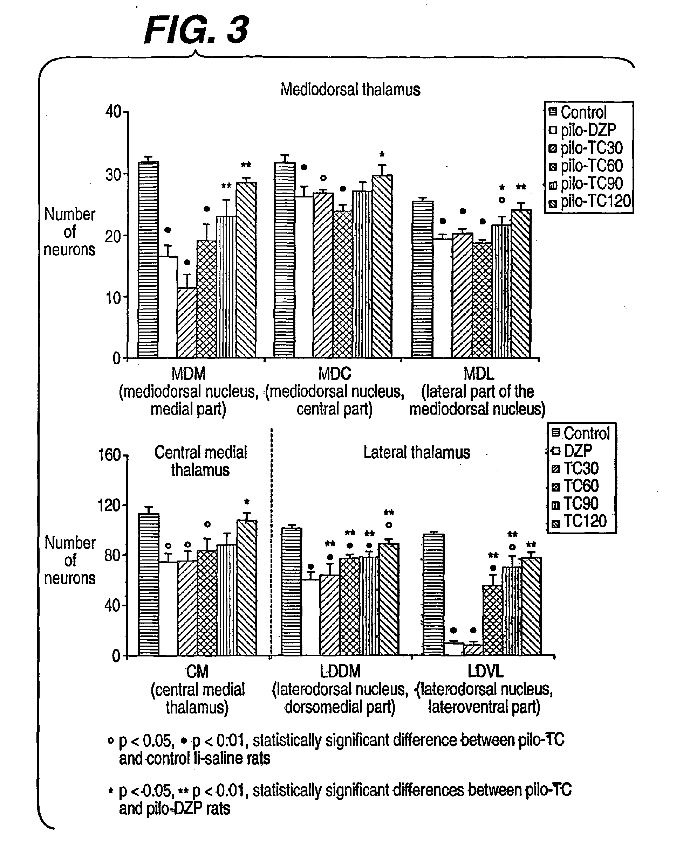

In the thalamus, neuronal loss was moderate in the mediodorsal central and lateral, the dorsolateral medial dorsal and in the central medial nuclei (18, 24, 40 and 34% drop out, respectively), more marked in the mediodorsal nucleus (49%) and major in the ventral lateral division of the dorsolateral nucleus (90%) (Table 1 and Figure 2). In the amygdala, neuronal loss was moderate in the medial ventral posterior nucleus (38%) and more marked in the basolateral and medial dorsal anterior nuclei (73 and 53% drop out, respectively). There was no neuronal damage in the central nucleus (Table 1 and Figure 3).

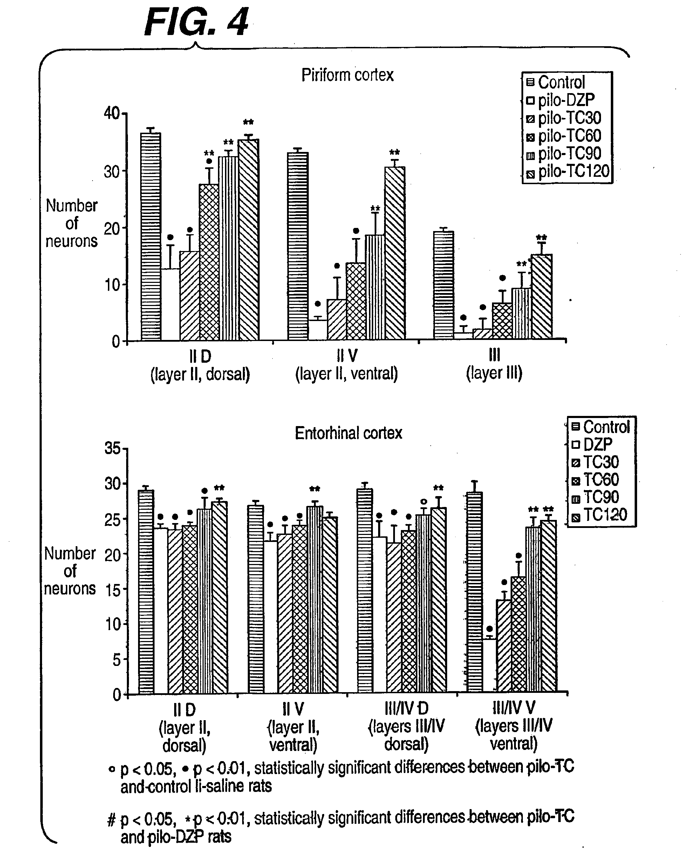

In the piriform cortex, neuronal loss was almost total in layer III (94%) which was no longer really visible and reached 66 and 89% in dorsal and ventral layer II, respectively in DZP rats compared to control saline-treated rats. In the dorsal entorhinal cortex, layers II and III-IV underwent slight damage (18 and 24%, respectively) and in ventral layers II and III/IV, damage reached 22 and 74%, respectively (Table 1 below and Figure 4).

**Table 1: Effects of increasing doses of Test Compound (TC) on the number of neuronal cell bodies in the hippocampus, thalamus, amygdala and cerebral cortex of rats subjected to li-pilo SE.**

| | Control (n=10) | pilo-DZP (n=7) | pilo-TC30 (n=8) | pilo-TC60 (n=11) | pilo-TC90 (n=10) | pilo-TC120 (n=8) |
|---|---|---|---|---|---|---|
| ***Hippocampus*** | | | | | | |
| CA1 area | 74.8 ± 1.5 | 10.9 ± 1.9** | 39.3 ± 4.4**°° | 31.9 ± 4.4**°° | 47.7 ± 6.6*° | 65.5 ± 2.9°° |
| CA3 area | 52.1 ± 2.7 | 31.3 ± 2.9** | 35.7 ± 1.8** | 31.6 ± 1.4** | 35.1 ± 2.9** | 39.8 ± 1.5** |
| Hilus | 96.4 ± 3.5 | 33.5 ± 3.0** | 33.0 ± 3.2** | 32.8 ± 3.3** | 37.5 ± 3.1** | 44.8 ± 2.9** |

| ***Thalamus*** | | | | | | |
|---|---|---|---|---|---|---|
| Mediodorsal medial | 31.9 ± 0.9 | 16.4 ± 1.9** | 11.5 ± 2.5** | 19.1 ± 2.6** | 23.1 ± 2.8°° | 28.6 ± 0.8°° |
| Mediodorsal central | 31.9 ± 1.2 | 26.3 ± 1.8** | 26.9 ± 0.6* | 24.1 ± 1** | 27.4 ± 1.5 | 29.9 ± 1.7° |
| Mediodorsal lateral | 25.9 ± 0.6 | 19.6 ± 0.8** | 20.5 ± 0.7** | 18.9 ± 0.6** | 22 ± 1.2*° | 24.4 ± 1.1°° |
| Dorsolateral, medial, dorsal | 102.2 ± 2.5 | 61 ± 6.3** | 64.2 ± 9.3**°° | 77.5 ± 3.9**°° | 79.4 ± 3.1**°° | 89.8 ± 3.7*° |
| Dorsolateral, ventral lateral | 97.8 ± 1.7 | 9.7 ± 2:5** | 8.8 ± 2.8** | 56.7 ± 8.7** | 71.8 ± 5.3°°* | 79.0 ± 4.7°° |
| Central medial | 113.1 ± 5.9 | 74.2 ± 7.4* | 75.6 ± 7.7* | 83.7 ± 9.6* | 88.2 ± 8.5 | 108.2 ± 6.6° |

| ***Amygdala*** | | | | | | |
|---|---|---|---|---|---|---|
| Basolateral | 46.7 ± 1.2 | 12.8 ± 5.3** | 27.3 ± 4.9**° | 27.8 ± 4.3**°° | 40.7 ± 1.6°° | 42.7 ± 1.3°° |
| Medial, dorsal anterior | 84.3 ± 3.8 | 40.0 ± 2.5** | 46.8 ± 5.0** | 58.4 ± 2.8**° | 72.2 ± 5.7°° | 80.2 ± 2.6°° |
| Medial, ventral posterior | 35.1 ± 1.7 | 21.8 ± 2.4** | 22.3 ± 1.8** | 26.2 ± 2.9** | 30.7 ± 3.7°° | 34.7 ± 1.7°° |

| ***Cerebral cortex*** | | | | | | |
|---|---|---|---|---|---|---|
| Piriform, layer II, dorsal | 36.6 ± 0.8 | 12.6 ± 4.2** | 15.7 ± 2.9** | 27.5 ± 2.8**°° | 32.4 ± 1.1°° | 35.2 ± 1.1°° |
| Piriform, layer II, ventral | 33.0 ± 0.8 | 3.6 ± 0.7** | 7.2 ± 3.8** | 13.7 ± 4.2** | 18.4 ± 4.0°° | 30.5 ± 1.3°° |
| Piriform, layer | 19.2 ± 0.7 | 1.2 ± 1.2** | 1.8 ± 1.8** | 6.4 ± 2.3** | 9 ± 3.0°° | 15 ± 2.2°° |
| Entorhinal, layer II, dorsal | 29 ± 0.6 | 23.5 ± 0.7** | 23.4 ± 0.6** | 23.9 ± 0.5** | 26.3 ± 0.9** | 27.3 ± 0.5°° |
| Entorhinal, layer II, ventral | 26.8 ± 0.7 | 21.7 ± 1.3** | 22.7 ± 0.9 | 23.3 ± 0.8** | 25.4 ± 1.1° | 25.1 ± 0.6 |
| Entorhinal, layer III/IV, dorsal | 29.2 ± 0.9 | 22.3 ± 0.5** | 22.3 ± 0.5** | 23.2 ± 0.8** | 26.7 ± 0.8* | 26.4 ± 0.70°° |
| Entorhinal,layer III/IV, ventral | 28.7 ± 1.7 | 7.7 ± 2.3** | 13.2 ± 1.9** | 16.5 ± 2.2** | 23.7 ± 1.5°° | 24.5 ± 1.4°° |

| | | | | | | |
|---|---|---|---|---|---|---|
| * p < 0.05, ** p< 0.01, statistically significant difference between pilo-TC and control li-saline rats ° p < 0.05, °° p< 0.01, statistically significant differences between pilo-TC and pilo-DZP rats | | | | | | |

In the hippocampus of TC-treated animals, cell loss was significantly reduced compared to DZP rats in CA1 pyramidal cell layer. This reduction was marked in TC30 , 60 or 90 rats (36-47% cell loss) and prominent in the TC120 group (12% cell loss). The differences were statistically significant at all TC doses (Table 1 and Figure 1). In the CA3 pyramidal layer, there was a tendency to a slight neuroprotection induced by Test Compound, only at the 120 mg/kg dose but the difference with the DZP group was not significant. In the dentate gyrus, the cell loss in the hilus was similar in the DZP and TC30 , 60 and 90 groups (61-66% drop out) and there was a slight tendency to reduced damage in the TC120 group (53% neuronal loss) compared to DZP animals (66% drop out). None of these differences was statistically significant.

In the thalamus, neuronal loss was similar in DZP and TC30 and TC60 rats. TC was significantly protective at the 60 mg/kg dose in the dorsolateral medial dorsal nucleus and at the two highest doses, 90 and 120 mg/kg in all thalamic nuclei, although the difference did not reach significance in the mediodorsal central and central medial nuclei in TC90 rats. In TC120 rats, neuronal drop out was considerably reduced compared to DZP rats. It ranged from 4-19% and the number of neurons was no longer significantly different from control animals, except in the dorsolateral medial dorsal nucleus (Table 1 and Figure 2). In the amygdala, TC was significantly protective at the 30 mg/kg dose in the basolateral nucleus and at the 60 mg dose, also in the medial dorsal anterior nucleus. At the highest dose, TC was largely neuroprotective; the number of neurons was no longer significantly different from the control level and reached 86-99% of the control level in all amygdala nuclei (Table 1 and Figure 3).

In the cerebral cortex, the treatment with TC did not significantly protect any cortical area compared to the DZP treatment at the dose of 30 mg/kg. At 60 mg/kg, TC significantly reduced neuronal loss only in layer II of the dorsal piriform cortex (25% drop out compared to 66% in the DZP group). At 90 and 120 mg/kg, TC significantly protected all three areas of the piriform cortex compared to the DZP treatment and at the highest dose of TC, 120 mg/kg, neuronal density reached 78-96% of control levels, even in piriform cortex, dorsal layer II and layer III where the neuronal population was almost totally depleted in the DZP group. In all layers of the dorsal and ventral entorhinal cortex, the two lowest doses of TC, 30 and 60 mg/kg did not afford any neuroprotection. The 90-mg/kg dose of TC significantly protected layers II and III/IV of the ventral entorhinal cortex (4 and 17% damage remaining in layers II and II/IV of the dorsal part and in layer II of the ventral part compared to 19 and 73% in the DZP group). At the highest dose of TC, 120 mg/kg, all parts of the entorhinal cortex, both dorsal and ventral were protected and the number of neurons in these areas was no longer significantly different from the level in controls (85-94% of neurons surviving compared to 27-81% in the DZP group). Latency to and frequency of recurrent seizures

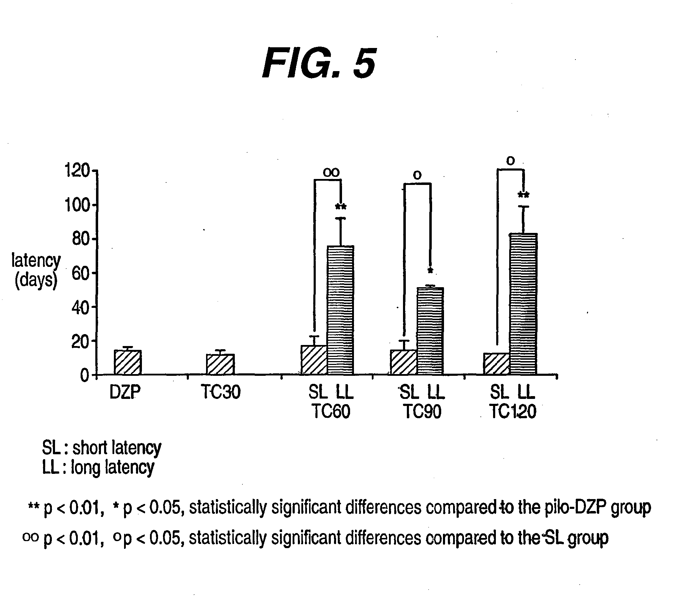

The latency to spontaneous seizures reached a mean value of 15.5 ± 2.3 days in the DZP group (14 rats) and was similar (11.6 ± 2.5 days) in the TC30 group (8 rats). At higher concentrations of TC, animals could be subdivided in subgroups with short and long latencies. A short latency was considered as any duration shorter than 40 days after SE. Some rats exhibited a latency to the first spontaneous seizure that was similar to that recorded in the DZP and TC groups but the number of rats exhibiting this short latency values progressively decreased with the increase in TC concentration. Thus at 30 mg/kg, 70% of the rats (7/10) had short latencies to seizures while at 90 and 120 mg/kg, this percentage reached 36% (4/11) and 11% (1/9), respectively (Table 2 below and Figure 5).

**Table 2: Effect of increasing doses of TC on the latency to spontaneous seizures.**

| Treatment | Number of animals | Latency to the first spontaneous seizure (days) | | | |
|---|---|---|---|---|---|
| DZP | 14 | 15.5 ± 2.34 | | | |
| pilo-TC30 | 8 | 11.6 ± 2.5 | | | |
| pilo-TC60 | 10 | **2 groups** | | | |
| | | Short latency (n=7) | | Long latency (n=3) | |
| | | 17.4 ± 5.4 | | 76.7 ± 15.6**°° | |
| pilo-TC90 | 11 | **3 groups** | | | |
| | | Short latency (n=4) | Long latency (n=2) | | Non epileptic (n=5) |
| | | 14.8 ± 5.7 | 52.0 ± 1.0*° | | 150** °° |
| | | **3 groups** | | | |
| pilo-TC120 | 9 | Short latency (n=1) | Long latency (n=4) | | Non epileptic (n=4) |
| | | 13.0 | 84.5 ± 16.7** °° | | 150** °° |

| | | | | | |
|---|---|---|---|---|---|
| ** p < 0.01, * p < 0.05, statistically significant differences compared to the pito-DZP group °° p < 0.01, ° p < 0.05, statistically significant differences compared to the short latency group | | | | | |

In the TC60, 90 and 120 groups, the mean value of the rats with long latencies was similar and ranged from 52 to 85 days. Finally, at the two highest doses of TC, we were able to identify a percentage of rats that did not develop any seizure over a duration of 150 days post-SE. The percentage of non-epileptic rats reached 45% at both doses of TC.

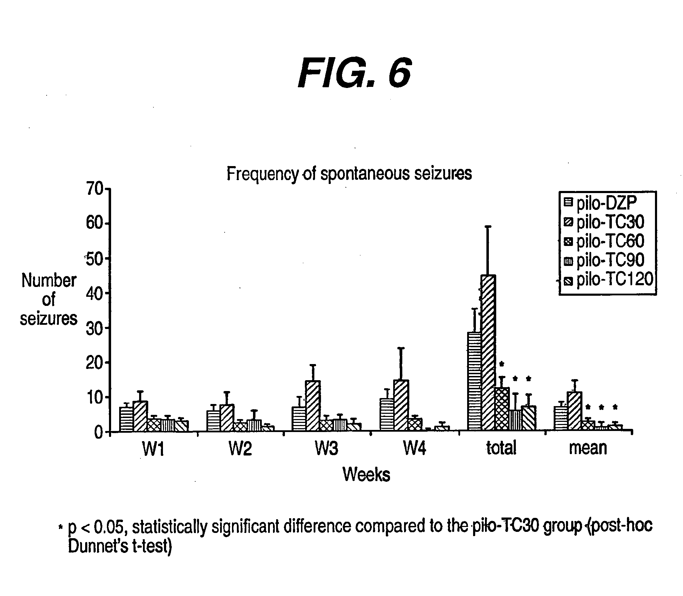

The frequency of spontaneous seizures was similar over the four weeks of recording. It showed a tendency to be higher in the DZP and TC30 groups while it was lower in the TC60, 90 and 120 groups (Figure 6). These differences did not reach statistical significance at the level of each individual weekly frequency but reached significance for the total or mean number of seizures over the four weeks.

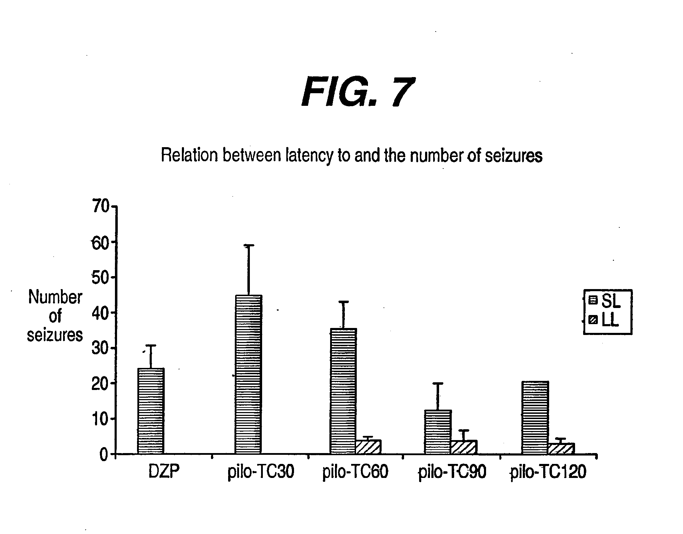

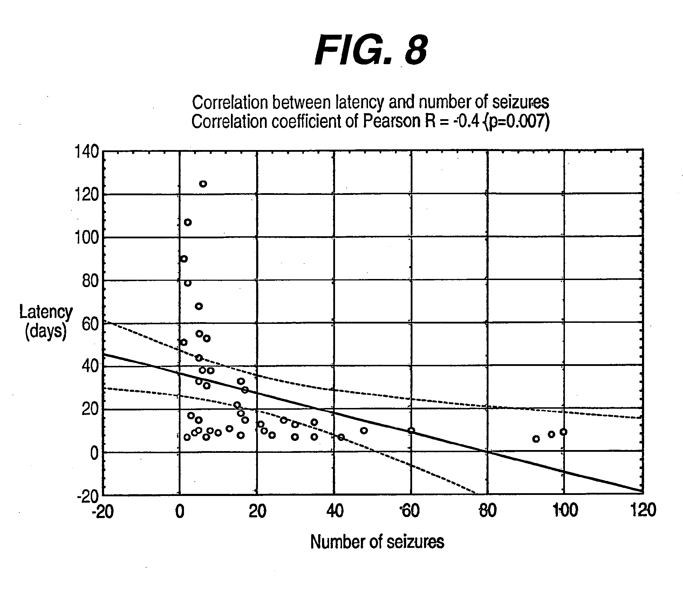

The number of seizures was also plotted according to the duration of the latency to the first spontaneous seizure. Animals with a short latency showed a tendency to display 2-3 times more seizures over the four weeks of recording than rats with a long latency period. No statistical analysis could be performed since the ANOVA did not show any significance, most likely because there was only one animal in the short latency subgroup of the TC120 animals (Figure 7). However, when all latency values were plotted against the number of seizures, there was a significant inverse correlation leading to a straight line with a correlation coefficient of - 0.4 (Figure 8).

To finalize this analysis, two more measurements will be performed. The first one is cell counting on the animals that were video recorded and followed for 2 months after the first spontaneous seizure or sacrificed at 5 months to study the potential correlation between the extent and location of brain damage and the occurrence of and/or latency to spontaneous seizures. The second one will be to perform a one-year follow-up of seizure occurrence in a group of rats to study whether or not the animals that we declare "non epileptic" at 5 months will remain seizure free.

The results of the present study show that a treatment with TC starting at 1 h after the onset of Li-pilo-induced SE has neuroprotective properties in the CA1 pyramidal cell layer of the hippocampus, and in all layers of the ventral and dorsal piriform and entorhinal cortex. TC protects also thalamus and amygdala nuclei. However, TC is not protective at the dose of 30 mg/kg, except in CA1, one thalamic and one amygdala nucleus. At the dose of 60 mg/kg, layer II of the dorsal piriform cortex and a second amygdala nucleus are also protected. At 90 and 120 mg/kg, the drug protects most cerebral regions studied, except hippocampal CA3 and the hilus of the dentate gyrus. The latter two structures plus the dorsolateral ventral dorsal thalamic nucleus are the only regions where the number of neurons remains significantly different from controls at the dose of 120 mg/kg TC. From these data, the extremely powerful neuroprotection properties of TC appear clearly. The molecule seems to prevent neuronal death in most regions belonging to the circuit of limbic epilepsy induced by Li-pilo, i.e., the hippocampus, thalamus, amygdala and parahippocampal cortices. These are all the regions in which we have detected MRI signal in the course of epileptogenesis in Li-pilo-treated rats (Roch C, Leroy C, Nehlig A, Namer IJ (2002a) Contribution of magnetic resonance imaging to the study of the lithium-pilocarpine model of temporal lobe epilepsy in adult rats. Epilepsia 43:325-335). The only two regions that are not efficiently protected by TC are CA3 pyramidal cell layer and the hilus of the dentate gyrus. The latter region undergoes rapid and massive cell damage (André V, Marescaux C, Nehlig A, Fritschy JM (2001) Alterations of the hippocampal GABAergic system contribute to the development of spontaneous recurrent seizures in the lithium-pilocarpine model of temporal lobe epilepsy. Hippocampus 11:452-468.; Roch C, Leroy C, Nehlig A, Namer IJ (2002a) Contribution of magnetic resonance imaging to the study of the lithium-pilocarpine model of temporal lobe epilepsy in adult rats. Epilepsia 43:325-335) and none of the neuroprotection that we used in previous studies have been able to protect this structure. We have also on the basis of earlier studies identified this structure as a key area in the initiation and maintenance of epileptic seizures in the Li-pilo model. (Dubé C, Marescaux C, Nehlig A (2000) A metabolic and neuropathological approach to the understanding of plastic changes occurring in the immature and adult rat brain during lithium-pilocarpine induced epileptogenesis. Epilepsia 41(Suppl 6):S36-S43)

Obviously, the present data demonstrate that epileptogenesis can be prevented even though damage remains quite marked in this area. Long-term cell counting on the group of animals that has been video recorded will be able to show whether or not the extent of damage in this region is critical for epileptogenesis in this model.

The treatment did not affect the latency to the first spontaneous seizure at the dose of 30 mg/kg. At the 3 higher doses, a percentage of animals developed epilepsy as fast as the DZP or TC30 rats but the relative importance of this subgroup was inversely related to the dose of TC used. Another subgroup, constant in size (2-4 animals per group) developed epilepsy after a 4-6 times longer latency while at the two highest doses of the drug, 4-5 rats had not become epileptic after 5 months, i.e. about 10 times the duration of the short latency and 2-3 times that of the long latency. This delay in the occurrence of epilepsy might correlate with the number of neurons protected in the basal cortices in the animals. This assumption is based on the fact that we noted some heterogeneity in the extent of neuroprotection in basal cortices of the animals subjected the short term neuronal counting at 14 days after SE. However, at the moment, we have not performed neuronal counting in the animals used for the study of epileptogenesis and therefore, no conclusion can be drawn on a potential relation between the number of neurons surviving in basal cortices and the rate or even occurrence of epileptogenesis.

The data obtained in the present study are in line with the previous study from this group reporting that the 60-mg/kg dose of Test compound (TC) protected the hippocampus and the basal cortices from neuronal damage and delayed the occurrence of recurrent seizures (see Example 1). They confirm that the protection of the basal cortices could be a key factor in inducing a disease modifying effect in the lithium-pilocarpine model of epilepsy. The key role of the basal cortices as initiators of the epileptic process was previously demonstrated by our group in the lithium-pilocarpine model (André V, Rigoulot MA, Koning E, Ferrandon A, Nehlig A (2003) Long-term pregabalin treatment protects basal cortices and delays the occurrence of spontaneous seizures in the lithium-pilocarpine model in the rat. Epilepsia 44:893-903; Roch C, Leroy C, Nehlig A, Namer IJ (2002a) Contribution of magnetic resonance imaging to the study of the lithium-pilocarpine model of temporal lobe epilepsy in adult rats. Epilepsia 43:325-335; Roch C, Leroy C, Nehlig A, Namer IJ (2002b) Predictive value of cortical injury for the development of temporal lobe epilepsy in P21-day-old rats: a MRI approach using the lithium-pilocarpine model. Epilepsia 43:1129-1136.

### References for Example 2

André V, Marescaux C, Nehlig A, Fritschy JM (2001) Alterations of the hippocampal GABAergic system contribute to the development of spontaneous recurrent seizures in the lithium-pilocarpine model of temporal lobe epilepsy. Hippocampus 11:452-468.
■ André V, Rigoulot MA, Koning E, Ferrandon A, Nehlig A (2003) Long-term pregabalin treatment protects basal cortices and delays the occurrence of spontaneous seizures in the lithium-pilocarpine model in the rat. Epilepsia 44:893-903.
■ Cavalheiro EA (1995) The pilocarpine model of epilepsy. Ital J Neurol Sci 16:33-37.
■ Dubé C, Marescaux C, Nehlig A (2000) A metabolic and neuropathological approach to the understanding of plastic changes occurring in the immature and adult rat brain during lithium-pilocarpine induced epileptogenesis. Epilepsia 41 (Suppl 6):S36-S43.
■ Dubé C, Boyet S, Marescaux C, Nehlig A (2001) Relationship between neuronal loss and interictal glucose metabolism during the chronic phase of the lithium-pilocarpine model of epilepsy in the immature and adult rat. Exp Neurol 167:227-241.
■ Paxinos G, Watson C (1986) The Rat Brain in Stereotaxic Coordinates, 2nd ed. Academic Press, San Diego.
■ Roch C, Leroy C, Nehlig A, Namer IJ (2002a) Contribution of magnetic resonance imaging to the study of the lithium-pilocarpine model of temporal lobe epilepsy in adult rats. Epilepsia 43:325-335.
■ Roch C, Leroy C, Nehlig A, Namer IJ (2002b) Predictive value of cortical injury for the development of temporal lobe epilepsy in P21-day-old rats: a MRI approach using the lithium-pilocarpine model. Epilepsia 43:1129-1136.
■ Turski L, Ikonomidou C, Turski WA, Bortolotto ZA, Cavalheiro EA (1989) Review: Cholinergic mechanisms and epileptogenesis. The seizures induced by pilocarpine: a novel experimental model of intractable epilepsy. Synapse 3:154-171.

The Test Compound (TC) referred to in the example below is the compound of Formula 7 and the same compound as in the other examples 1 and 2 above.

### EXAMPLE 3

The purpose of this study was to assess the pharmacokinetics (PK) of Test Compound (TC) following single and repeated oral administration in healthy adult men at clinically relevant doses

### METHODS:

Two single-center, placebo-controlled, double-blind, ascending-dose studies were conducted in healthy men ≥18 and ≥45 yrs. In study 1 (N=70), subjects were randomly assigned to a single dose of Test Compound (TC) or placebo. Escalated doses were received as 100, 250, 400, 750, 1000, 1250, and 1500 mg. PK parameters were estimated from plasma and urine samples collected up to 3 days post dose. Study 2 (N=53) evaluated the PK of repeated doses of Test Compound (TC) in 4 dose groups (100, 250, 500, or 750 mg). Within each group, 12 subjects were assigned to q12h treatment with drug or placebo for 1 wk and were crossed over after a 14-day washout period. PK parameters were estimated from plasma and urine samples on days 1 and 7.

### RESULTS:

Single dose: Test Compound (TC) was rapidly absorbed following oral administration. Cₘₐₓ and AUC_{0-∞} increased in proportion to dose over the range of 100-1500 mg. Mean tₘₐₓ ranged from 1.3-2.7 h. Mean t_{1/2} (11.5 - 13.9 h), CUF (2.87-3.67 Uh), and Vd/F (52.1-66.3 Uh) values were similar for all 7 dose groups.

Repeated doses: Plasma concentrations of Test Compound (TC) reached steady state after 3-4 days as predicted from its single-dose half-life. Mean tₘₐₓ occurred 1.3-1.8 h after dosing. The mean t_{1/2} (11.9-12.8 h) and CUF (3.40-3.78 Uh) values at steady state were comparable to the PK parameters following single-dose administration on day 1 and in study 1. Steady-state Cₘₐₓ and AUC₀₋₁₂ increased in proportion to the dose.

As expected, there was a moderate degree of Test Compound (T-C) accumulation; Cₘₐₓ and AUC₀₋₁₂ were about two-fold higher on day 7 vs. day 1 (P<0.001). Mean CL_{R} estimates for Test Compound (TC) were <5% of the mean oral clearance, suggesting non-renal clearance as the primary mechanism for Test Compound (TC) elimination.

### CONCLUSIONS:

Test Compound (TC) exhibited linear PK after single (100-1500mg) and repeated (100-750 mg bid) doses. It was rapidly absorbed and had a mean elimination half life of 11.5-13.9 h, allowing bid -dosing. Following q12h administration, Test Compound (TC) accumulated two-fold and was primarily cleared by a non-renal pathway.

### EXAMPLE 4

### Prophetic example of a treatment regimen with Test Compound (TC):

A 23 year old male soldier is admitted to the hospital with a penetrating head wound from a bomb fragment. The fragment entered the right frontal lobe of the brain and penetrated approximately one inch into the brain. The fragment is removed surgically and the patient recovers well with minimal neurological dysfunction. The patient does not have any personal or family history of a seizure disorder and does not manifest any evidence of a seizure disorder following surgery and EEG's are negative for seizure activity. The patient's physician immediately initiates a treatment regimen with Test Compound (TC) at a dose of 500 mg twice a day to prevent development of a seizure disorder as a result of the injury. The prophylactic treatment is continued for one year and at follow up the patient shows no evidence of development of a seizure disorder. The patient is followed for an additional year and shows no evidence of development of a seizure disorder.

### EXAMPLE 5

### Prophetic example of a treatment regimen with Test Compound (TC):

A 37-year-old female is admitted to the hospital after suffering blunt head trauma in a car accident. The patient has no history of any type of seizure disorder and personal and family medical history are essentially negative. The patient's head struck the dashboard of the car and she lost conciseness for 30 minutes after the accident. A CT scan shows a small contusion in the left frontal region of the brain and the patient is diagnosed as having blunt head trauma with a concussion. The patient's physician is concerned about the possible future development of a seizure disorder as a result of the injury to the patient's frontal lobe. The patient's physician immediately initiates a treatment regimen with Test Compound (TC) at a dose of 300 mg twice a day in order to prevent the development of a seizure disorder. The patient is followed for one year and shows no evidence of seizure development. The dose of Test Compound is gradually tapered and stopped and the patient remains seizure free on follow up two years later.

### EXAMPLE 6

### Prophetic example of a treatment regimen with Test Compound (TC):

A 74 year old female is admitted to the hospital following an episode at home when she developed right-sided weakness and inability to speak. An MRI shows a left middle cerebral artery stroke. Patient has no personal or family history of seizures or other neurological problems. In addition to routine supportive care the patient's physician initiates a regimen of Test Compound (TC) at a dose of 250 mg twice a day to prevent the development of a seizure disorder in the post stroke recovery period. The patient is seen in follow one year later and has no evidence of development of a seizure disorder and is gradually tapered off the medication. The patient continues to be seizure free on follow up in two years.

### EXAMPLE 7

### Prophetic example of a treatment regimen with Test Compound (TC):

A 7 year old boy previously in good health is seen in his physician's office after having had repeated seizures at home in the context of a fever of 105 degrees secondary to a viral upper respiratory infection. The patient has recovered from the viral infection and shows no signs of having a seizure disorder. The physician diagnoses febrile convulsions. The boy weighs 27 kilograms and the physician decides to initiate a regimen of Test Compound (TC) at a dose 7.1 mg/kg/day and therefore begins the boy on 100 mg twice a day to prevent the development of a seizure disorder. The patient is seen in follow up and is seizure free at one year. The medication is continued for two years and tapered off. Patient shows no evidence of a seizure disorder on follow up at three years.

### EXAMPLE 8

### Prophetic example of a treatment regimen with Test Compound (TC):

A 47 yr old male is admitted to the hospital for excision of a right prefrontal AVM. The patient has no personal or family history of seizure disorder. Prior to the neurosurgical procedure the patient's physician initiates a regimen of Test Compound at a dose of 200 mg twice a day to minimize the risk of development of a seizure disorder post surgery. The patient is seen in follow up one year after the procedure and the medication is tapered and stopped. The patient continues to do well and shows no evidence of developing a seizure disorder on follow up three years later.

The present invention is not to be limited in terms of the particular embodiments or examples described in this application, which are intended as single illustrations of individual aspects of the invention. Many modifications and variations of this invention can be made without departing from its scope, as will be apparent to those skilled in the art. Functionally equivalent methods and combinations within the scope of the invention, in addition to those enumerated herein will be apparent to those skilled in the art from the foregoing description, examples and accompanying drawings. Such modifications and variations are intended to fall within the scope of the appended claims. The present invention is to be limited only by the terms of the appended claims.

## Claims

1. A compound, or a pharmaceutically acceptable salt or ester thereof, selected from the group consisting of Formula (I) and Formula (II): 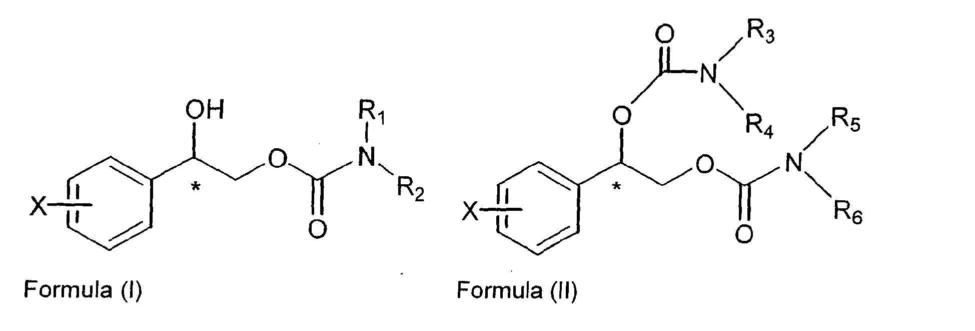 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano) for use in treating preventing, reversing, arresting or inhibiting epileptogenesis in a human subject who is at risk of developing epilepsy or a seizure related disorder but does not have epilepsy or clinical evidence of seizures.

2. The compound as claimed in claim 1 wherein X is chlorine.

3. The compound as claimed in claim 1 wherein X is substituted at the ortho position of the phenyl ring.

4. The compound as claimed in claim 1 wherein R₁, R₂, R₃, R₄, R₅ and R₆ are selected from hydrogen.

5. An enantiomer, or a pharmaceutically acceptable salt or ester thereof, selected from the group consisting of Formula (I) and Formula (II) or enantiomeric mixture wherein one enantiomer selected from the group consisting of Formula (I) and Formula (II) predominates: 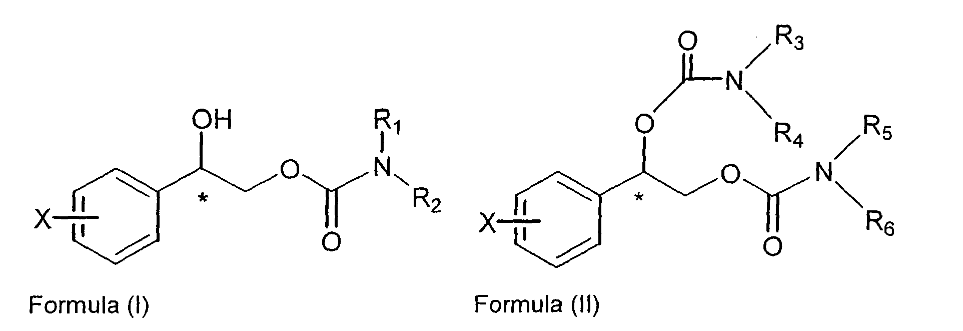 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano) for use in treating preventing, reversing, arresting or inhibiting epileptogeneis in a human subject who is at risk of developing epilepsy or a seizure related disorder but does not have epilepsy or clinical evidence of seizures.

6. The enantiomer as claimed in claim 5 wherein X is chlorine.

7. The enantiomer as claimed in claim 5 wherein X is substituted at the ortho position of the phenyl ring.

8. The enantiomer as claimed in claim 5 wherein R₁, R₂, R₃, R₄, R₅ and R₆ are selected from hydrogen.

9. The enantiomer as claimed in claim 5 wherein one enantiomer selected from the group consisting of Formula (I) and Formula (II) predominates to the extent of about 90% or greater.

10. The enantiomer as claimed in claim 5 wherein one enantiomer selected from the group consisting of Formula (I) and Formula (II) predominates to the extent of about 98% or greater.

11. The enantiomer as claimed in claim 5 wherein the enantiomer selected from the group consisting of Formula (I) and Formula (II) is an enantiomer selected from the group consisting of Formula (Ia) and Formula (IIa): 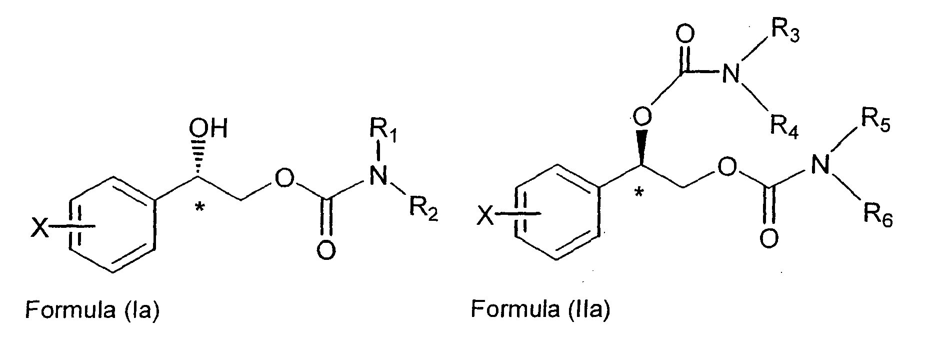 wherein
phenyl is substituted at X with one to five halogen atoms selected from the group consisting of fluorine, chlorine, bromine and iodine; and,
R₁, R₂, R₃, R₄, R₅ and R₆ are independently selected from the group consisting of hydrogen and C₁-C₄ alkyl; wherein C₁-C₄ alkyl is optionally substituted with phenyl (wherein phenyl is optionally substituted with substituents independently selected from the group consisting of halogen, C₁-C₄ alkyl, C₁-C₄ alkoxy, amino, nitro and cyano).

12. The enantiomer as claimed in claim 11 wherein X is chlorine.

13. The enantiomer as claimed in claim 11 wherein X is substituted at the ortho position of the phenyl ring.

14. The enantiomer as claimed in claim 11 wherein R₁, R₂, R₃, R₄, R₅ and R₆ are selected from hydrogen.

15. The enantiomer as claimed in claim 11 wherein one enantiomer selected from the group consisting of Formula (Ia) and Formula (IIa) predominates to the extent of about 90% or greater.

16. The enantiomer as claimed in claim 11 wherein one enantiomer selected from the group consisting of Formula (Ia) and Formula (IIa) predominates to the extent of about 98% or greater.

17. The enantiomer as claimed in claim 5 wherein the enantiomer selected from the group consisting of Formula (I) and Formula (II) is an enantiomer selected from the group consisting of Formula (Ib) and Formula (IIb) or a pharmaceutically acceptable salt or ester form thereof:

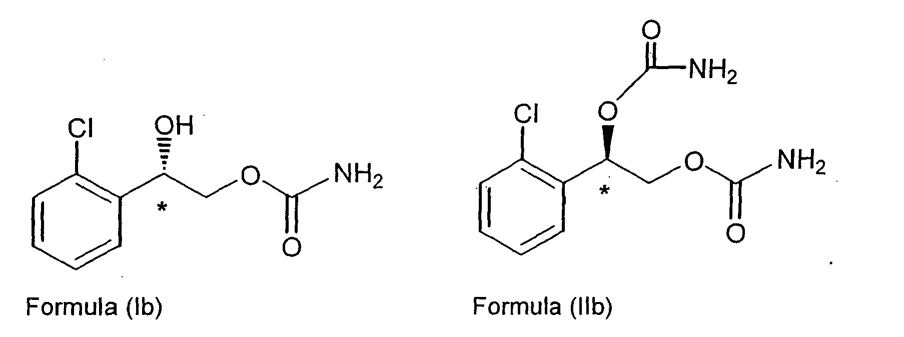

18. The enantiomer as claimed in claim 17 wherein one enantiomer selected from the group consisting of Formula (Ib) and Formula (IIb) predominates to the extent of about 90% or greater.

19. The enantiomer as claimed in claim 17 wherein one enantiomer selected from the group consisting of Formula (Ib) and Formula (IIb) predominates to the extent of about 98% or greater.

20. The compound or enantiomer, as claimed in claims 1 or 5 wherein the predisposing factor(s) rendering the patient at risk of developing epilepsy or a seizure related disorder is selected from the group consisting of: injury or trauma of any kind to the CNS; neurosurgical procedures, activities that risk CNS injury, e.g., combat activities, auto or horse racing and contact sports including boxing; spinal cord trauma; infections of the CNS; anoxia; stroke (CVAs); history of Transient Ischemic Attacks (TIA's); carotid stenosis; history of athererosclerotic vessel disease; history of pulmonary emboli; peripheral vascular disease; autoimmune diseases affecting the CNS, e.g., lupus; birth injures, e.g., perinatal asphyxia; cardiac arrest; therapeutic or diagnostic vascular surgical procedures, e.g., carotid endarterectomy or cerebral angiography; hypotension; injury to the CNS from emboli, hyper or hypo perfusion; hypoxia; known genetic predisposition to disorders known to respond to AEGDs; space occupying lesions of the CNS; brain tumors, e.g., glioblastomas; bleeding or hemorrhage in or surrounding the CNS, e.g., intracerebral bleeds or subdural hematomas; brain edema; febrile convulsions; hyperthermia; exposure to toxic or poisonous agents; drug intoxication or withdrawal, e.g. cocaine, methamphetamine or alcohol; family history of; seizure disorders or an epilepsy related seizure like neurological disorder or seizure related disorder, history of status epilepticus; current treatment with medications that lower seizure threshold, e.g., lithium carbonate, thorazine or clozapine; evidence from surrogate markers or biomarkers that the patient is in need of treatment with an anti-epileptogenic drug, e.g. MRI scan showing hippocampal sclerosis, elevated serum levels of neuronal degradation products, elevated levels of ciliary neurotrophic factor (CNTF) or an EEG suggestive of a seizure disorder or an epilepsy related seizure like neurological disorder.

21. The compound or enantiomer as claimed in claim 20 wherein the predisposing factor(s) rendering the patient at risk of developing epilepsy or a seizure related disorder is selected from the group consisting of: closed or penetrating head trauma; neurosurgical procedures, carotid stenosis, stroke or other cerebral-vascular accident (CVA); status epilepticus and space occupying lesions of the CNS..

22. The compound or enantiomer as claimed in claim 21 wherein the said predisposing factor(s) are closed head trauma or penetrating head trauma or a neurosurgical procedure.

23. The compound or enantiomer as claimed in claim 21 wherein the said predisposing factor(s) are; stroke, other cerebral-vascular accident (CVA), presence of carotid stenosis or Transient Ischemic Attack's.

24. The compound or enantiomer as claimed in claim 23 wherein the said predisposing factor is status epilepticus.

25. The compounds or enantiomers as claimed in claims 1 or 5 wherein said compound (or enantiomer) or a pharmaceutically acceptable salt or ester thereof is administered in combination administration with one or more other compounds or therapeutic agents.

26. The compounds or enantiomers as claimed in claim 25 wherein the said one or more other compounds or therapeutic agents are selected from the group consisting of compounds that have one or more of the following properties: antioxidant activity; NMDA receptor antagonism; ability to augment endogenous GABA inhibition; NO synthase inhibitor activity; iron binding ability, e.g., an iron chelator; calcium binding ability, e.g., a Ca (II) chelator; zinc binding ability, e.g., a Zn (II) chelator; the ability to block sodium or calcium ion channels; the ability to open potassium or chloride ion channels, therapeutic agents useful in the treatment of Substance Abuse.

27. The compounds or enantiomers as claimed in claim 25 wherein the said one or more compounds are selected from the group consisting of anti-epileptic drugs (AEDs).

28. The compounds or enantiomers as claimed in claim 27 wherein the said anti-epileptic drug (AED) is selected from the group consisting of; carbamazepine, clobazam, clonazepam, ethosuximide, felbamate, gabapentin, lamotigine, levetiracetam, oxcarbazepine, phenobarbital, phenytoin, pregabalin, primidone, retigabine, talampanel, tiagabine, topiramate, valproate, vigabatrin, zonisamide, benzodiazepines, barbiturates or sedative hypnotics.

29. The compound or enantiomer as claimed in claims 1 or 5 wherein the therapeutically effective amount is from about 5.7 mg/Kg/day to about 42.9 mg/Kg/day.

30. The compound or enantiomer, as claimed in claims 1 or 5, wherein the said therapeutic amount is progressively decreased as the treatment of the epileptogenic process progresses in the said patient.

31. The compound or enantiomer, as claimed in claims 25, 26, 27 or 28 wherein the amount of the said one or more other compounds or therapeutic agents administered in combination with the said compound (or enantiomer) or a pharmaceutically acceptable salt or ester thereof is progressively decreased as the treatment of the epileptogenic process progresses in the said patient.

32. The compound or enantiomer as claimed in claims 1 or 5 wherein the dose administered is from about 6.4 mg/Kg/day to about 35.7 mg/Kg/day.

33. The compound or enantiomer as claimed in claims 1 or 5 wherein the dose administered is from about 7.1 mg/Kg/day to about 28.6 mg/Kg/day.

34. The compound or enantiomer as claimed in claims 1 or 5 wherein the dose administered is from about 7.9 mg/Kg/day to about 21.4 mg/Kg/day.

35. The compound or enantiomer as claimed in claims 1 or 5 wherein the dose administered is from about 8.6 mg/Kg/day to about 17.1 mg/Kg/day.

36. The compound or enantiomer, as claimed in claims 1 or 5, wherein the dose administered is from about 400 mg/day to about 3000 mg/day.

37. The compound or enantiomer, as claimed in claims 1 or 5, wherein the dose administered is from about 450 mg/day to about 2500 mg/day.

38. The compound or enantiomer, as claimed in claims 1 or 5, wherein the dose administered is from about 500 mg/day to about 2000 mg/day.

39. The compound or enantiomer, as claimed in claims 1 or 5, wherein the dose administered is from about 550 mg/day to about 1500 mg/day.

40. The compound or enantiomer, as claimed in claims 1 or 5, wherein the dose administered is from about 600 mg/day to about 1200 mg/day.

## Patentansprüche

1. Verbindung oder pharmazeutisch verträgliches/verträglicher Salz oder Ester davon, ausgewählt aus der Gruppe, bestehend aus Formel (I) und Formel (II): 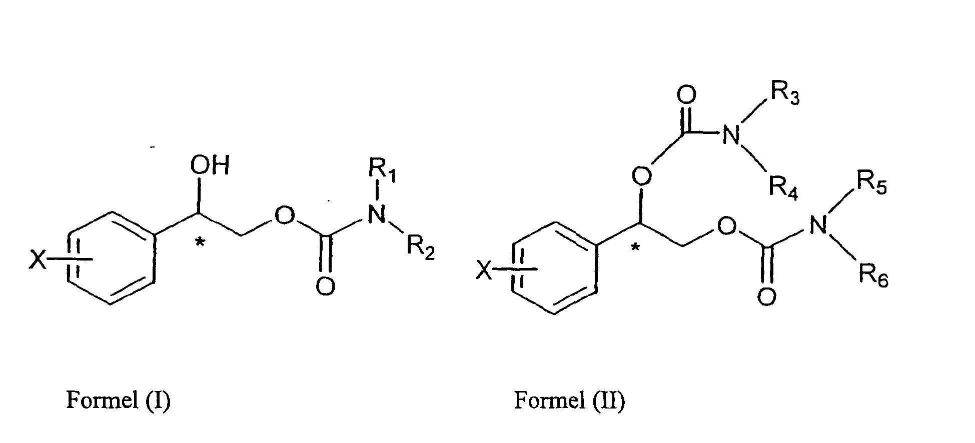 worin
Phenyl bei X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl gegebenenfalls mit Phenyl substituiert ist (wobei Phenyl gegebenenfalls mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano), zur Verwendung bei Behandlung, Verhinderung, Umkehrung, Stoppen oder Hemmen von Epileptogenese bei einem menschlichen Patienten, der unter dem Risiko steht, Epilepsie oder eine mit Anfällen zusammenhängende Störung zu entwickeln, aber keine Epilepsie oder klinische Evidenz von Anfällen hat.

2. Verbindung nach Anspruch 1, wobei X Chlor ist.

3. Verbindung nach Anspruch 1, wobei X an der ortho-Position des Phenylrings substituiert ist.

4. Verbindung nach Anspruch 1, wobei R₁, R₂, R₃, R₄, R₅ und R₆ ausgewählt sind aus Wasserstoff.

5. Enantiomer oder pharmazeutisch verträgliches/verträglicher Salz oder Ester davon, ausgewählt aus der Gruppe, bestehend aus Formel (I) und Formel (II), oder Enantiomerenmischung, in der ein Enantiomer, das ausgewählt ist aus der Gruppe, bestehend aus Formel (I) und Formel (II), überwiegt: 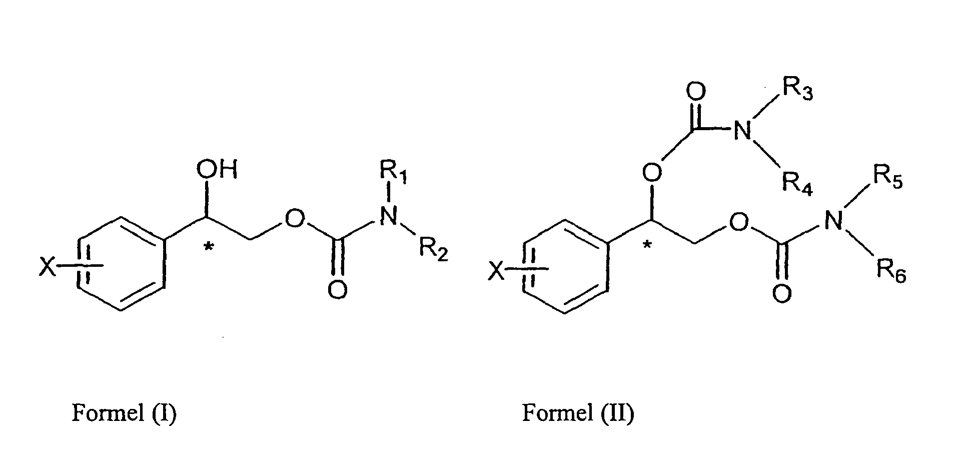 worin
Phenyl bei X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl gegebenenfalls mit Phenyl substituiert ist (wobei Phenyl gegebenenfalls mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano), zur Verwendung bei Behandlung, Verhinderung, Umkehrung, Stoppen oder Hemmen von Epileptogenese bei einem menschlichen Patienten, der unter dem Risiko steht, Epilepsie oder eine mit Anfällen zusammenhängende Störung zu entwickeln, aber keine Epilepsie oder klinische Evidenz von Anfällen hat.

6. Enantiomer nach Anspruch 5, wobei X Chlor ist.

7. Enantiomer nach Anspruch 5, wobei X an der ortho-Position des Phenylrings substituiert ist.

8. Enantiomer nach Anspruch 5, wobei R₁, R₂, R₃, R₄, R₅ und R₆ ausgewählt sind aus Wasserstoff.

9. Enantiomer nach Anspruch 5, wobei ein Enantiomer, das ausgewählt ist aus der Gruppe, bestehend aus Formel (I) und Formel (II), bis zum Umfang von etwa 90% oder mehr überwiegt.

10. Enantiomer nach Anspruch 5, wobei ein Enantiomer, das ausgewählt ist aus der Gruppe, bestehend aus Formel (I) und Formel (II), bis zum Umfang von etwa 98% oder mehr überwiegt.

11. Enantiomer nach Anspruch 5, wobei das Enantiomer, das ausgewählt ist aus der Gruppe, bestehend aus Formel (I) und Formel (II), ein Enantiomer ist, das ausgewählt ist aus der Gruppe, bestehend aus Formel (Ia) und Formel (IIa): 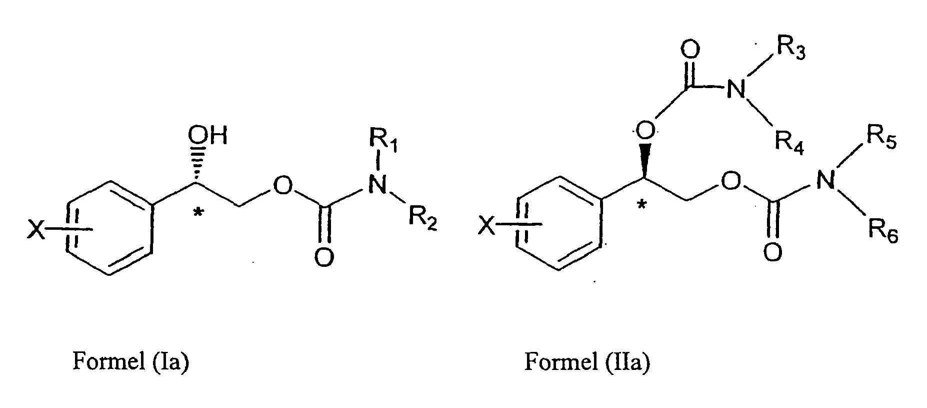 worin
Phenyl bei X mit einem bis fünf Halogenatomen substituiert ist, die ausgewählt sind aus der Gruppe, bestehend aus Fluor, Chlor, Brom und Iod; und
R₁, R₂, R₃, R₄, R₅ und R₆ unabhängig ausgewählt sind aus der Gruppe, bestehend aus Wasserstoff und C₁-C₄-Alkyl; wobei C₁-C₄-Alkyl gegebenenfalls mit Phenyl substituiert ist (wobei Phenyl gegebenenfalls mit Substituenten substituiert ist, die unabhängig ausgewählt sind aus der Gruppe, bestehend aus Halogen, C₁-C₄-Alkyl, C₁-C₄-Alkoxy, Amino, Nitro und Cyano).

12. Enantiomer nach Anspruch 11, wobei X Chlor ist.

13. Enantiomer nach Anspruch 11, wobei X an der ortho-Position des Phenylrings substituiert ist.

14. Enantiomer nach Anspruch 11, wobei R₁, R₂, R₃, R₄, R₅ und R₆ ausgewählt sind aus Wasserstoff.

15. Enantiomer nach Anspruch 11, wobei ein Enantiomer, das ausgewählt ist aus der Gruppe, bestehend aus Formel (Ia) und Formel (IIa), bis zum Umfang von etwa 90% oder mehr überwiegt.

16. Enantiomer nach Anspruch 11, wobei ein Enantiomer, das ausgewählt ist aus der Gruppe, bestehend aus Formel (Ia) und Formel (IIa), bis zum Umfang von etwa 98% oder mehr überwiegt.

17. Enantiomer nach Anspruch 5, wobei das Enantiomer, das ausgewählt ist aus der Gruppe, bestehend aus Formel (I) und Formel (II), ein Enantiomer ist, das ausgewählt ist aus der Gruppe, bestehend aus Formel (Ib) und Formel (IIb), oder einer pharmazeutisch verträglichen Salz- oder Esterform davon:

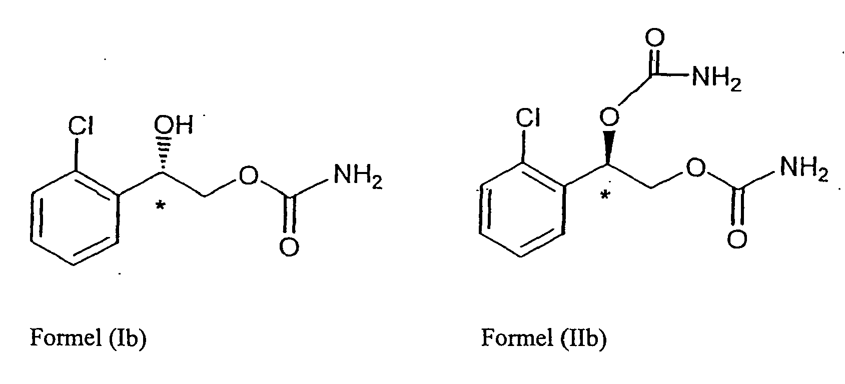

18. Enantiomer nach Anspruch 17, wobei ein Enantiomer, das ausgewählt ist aus der Gruppe, bestehend aus Formel (Ib) und Formel (IIb), bis zum Umfang von etwa 90% oder mehr überwiegt.

19. Enantiomer nach Anspruch 17, wobei ein Enantiomer, das ausgewählt ist aus der Gruppe, bestehend aus Formel (Ib) und Formel (IIb), bis zum Umfang von etwa 98% oder mehr überwiegt.

20. Verbindung oder Enantiomer nach den Ansprüchen 1 oder 5, wobei der (die) prädisponierende(n) Faktor(en), der (die) den Patienten dem Risiko unterwirft (unterwerfen), Epilepsie oder eine mit Anfällen zusammenhängende Störung zu entwickeln, ausgewählt ist (sind) aus der Gruppe, bestehend aus: Verletzung oder Trauma jeglicher Art des ZNS; neurochirurgischen Verfahren, Aktivitäten, die ZNS-Verletzungen riskieren, z.B. Kampfaktivitäten, Auto- oder Pferderennen und Kontaktsportarten, einschließlich Boxen; Rückenmarktrauma; Infektionen des ZNS; Anoxie; Schlaganfall (CVAs); Geschichte transitorischer ischämischer Attacken (TIAs); Karotisstenose; Geschichte atherosklerotischer Gefäßerkrankung; Geschichte von Lungenembolien; peripherer Gefäßerkrankung; Autoimmunerkrankungen, die das ZNS befallen, z.B. Lupus; Geburtsverletzungen, z.B. perinataler Asphyxie; Herzstillstand; therapeutischen oder diagnostischen gefäßchirugischen Verfahren, z.B. Karotisendarterektomie oder zerebraler Angiographie; niedrigem Blutdruck; Verletzung des ZNS durch Embolien, Über- oder Unterperfusion; Hypoxie; bekannter genetischer Prädisposition für Störungen, von denen bekannt ist, dass sie auf AEGDs ansprechen; raumfordernden Läsionen des ZNS; Hirntumoren, z.B. Glioblastomen; Blutung oder Hämorrhag in oder in der Umgebung des ZNS, z.B. interzerebralen Blutungen oder subduralen Hämatomen; Himödem; Fieberkrämpfen; Hyperthermie; Einwirkung von toxischen oder giftigen Mitteln; Substanzintoxikation oder -entzug, z.B. Kokain, Methamphetamin oder Alkohol; Familiengeschichte von Anfallsstörungen oder einer mit Epilepsie zusammenhängenden abfallsähnlichen neurologischen Störung oder mit Anfällen zusammenhängender Störung, Geschichte von Status epilepticus; laufender Behandlung mit Medikationen, die den Anfallsschwellenwert senken, z.B. Lithiumcarbonat, Thorazin oder Clozapin; Evidenz von Surrogatmarkern oder Biomarkern, dass der Patient einer Behandlung mit einem antiepileptogenen Arzneistoff bedarf, z.B. MRI-Scan, der Hippokampusklerose, erhöhte Serumspiegel neuronaler Abbauprodukte, erhöhte Spiegel an ciliärem neurotrophen Faktor (CNTF) zeigt, oder ein EEG, das eine Anfallsstörung oder eine mit Epilepsie zusammenhängenden anfallsähnliche neurologische Störung nahelegt.

21. Verbindung oder Enantiomer nach Anspruch 20, wobei der (die) prädisponierende(n) Faktor(en), der (die) den Patienten dem Risiko unterwirft (unterwerfen), Epilepsie oder eine mit Anfällen zusammenhängende Störung zu entwickeln, ausgewählt ist (sind) aus der Gruppe, bestehend aus: geschlossenem oder penetrierendem Kopftrauma; neurochirurgischen Verfahren, Karotisstenose, Schlaganfall oder anderem zerebrovaskulärem Insult (CVA); Status epilepticus und raumfordernden Läsionen des ZNS.

22. Verbindung oder Enantiomer nach Anspruch 21, wobei der (die) prädisponierende(n) Faktor(en) geschlossenes Kopftrauma oder penetrierendes Kopftrauma oder ein neurochirugisches Verfahren ist (sind).

23. Verbindung oder Enantiomer nach Anspruch 21, wobei der (die) prädisponierend(n) Faktor(en) Schlaganfall, anderer zerebrovaskulärer Insult (CVA), Vorliegen von Karotisstenose oder transitorische ischämische Attacken ist (sind).

24. Verbindung oder Enantiomer nach Anspruch 23, wobei der prädisponierende Faktor Status epilepticus ist.

25. Verbindungen oder Enantiomere nach den Ansprüchen 1 oder 5, wobei die Verbindung (oder das Enantiomer) oder ein pharmazeutisch verträgliches/verträglicher Salz oder Ester davon in Kombinationsverabreichung mit einem oder mehreren anderen Verbindungen oder therapeutischen Mitteln verabreicht wird.

26. Verbindungen oder Enantiomere nach Anspruch 25, wobei die eine oder mehreren anderen Verbindungen oder therapeutischen Mittel ausgewählt sind aus der Gruppe, bestehend aus Verbindungen, die eine oder mehrere der folgenden Eigenschaften besitzen: antioxidierende Aktivität; NMDA-Rezeptor-Antagonismus; Fähigkeit, endogene GABA-Hemmung zu erhöhen; NO-Synthasehemmer-Aktivität; Eisenbindungsfähigkeit, z.B. ein Eisen-Chelatbildner; Calciumbindungsfähigkeit, z.B. ein Ca(II)-Chelatbildner; Zinkbindungsfähigkeit, z.B. ein Zn(II)-Chelatbildner; die Fähigkeit, Natrium- oder Calciumionenkanäle zu blockieren; die Fähigkeit, Kalium- oder Chloridionenkanäle zu öffnen, therapeutische Mittel, die bei der Behandlung von Substanzmissbrauch nützlich sind.

27. Verbindungen oder Enantiomere nach Anspruch 24, wobei das eine oder die mehreren Verbindungen ausgewählt sind aus der Gruppe, bestehend aus antiepileptischen Arzneistoffen (AEDs).

28. Verbindungen oder Enantiomere nach Anspruch 27, wobei der antiepileptische Arzneistoff (AED) ausgewählt ist aus der Gruppe, bestehend aus Carbamezepin, Clobazam, Clonazepam, Ethosuximid, Felbamat, Gabapentin, Lamotigin, Levetiracetam, Oxcarbazepin, Phenobarbital, Phenytoin, Pregabalin, Primidon, Retigabin, Talampanel, Tiagabin, Topiramat, Valproat, Vigabatrin, Zonisamid, Benzodiazepinen, Barbituraten oder sedativen Hypnotika.

29. Verbindung oder Enatiomer nach den Ansprüchen 1 oder 5, wobei die therapeutisch wirksame Menge von etwa 5,7 mg/kg/Tag bis etwa 42,9 mg/kg/Tag beträgt.

30. Verbindung oder Enantiomer nach den Ansprüchen 1 oder 5, wobei die therapeutische Menge progressiv gesenkt wird, wenn die Behandlung des epileptogenen Prozesses im Patienten voranschreitet.

31. Verbindung oder Enantiomer nach den Ansprüchen 25, 26, 27 oder 28, wobei die Menge der einen oder mehreren anderen Verbindungen oder therapeutischen Mittel, die in Kombination mit der Verbindung (oder dem Enantiomer) oder einem pharmazeutisch verträglichen Salz oder Ester davon verabreicht werden, progressiv gesenkt wird, wenn die Behandlung des epileptogenen Prozesses im Patienten voranschreitet.

32. Verbindung oder Enantiomer nach den Ansprüchen 1 oder 5, wobei die verabreichte Dosis von etwa 6,4 mg/kg/Tag bis etwa 35,7 mg/kg/Tag beträgt.

33. Verbindung oder Enantiomer nach den Ansprüchen 1 oder 5, wobei die verabreichte Dosis von etwa 7,1 mg/kg/Tag bis etwa 28,6 mg/kg/Tag beträgt.

34. Verbindung oder Enantiomer nach den Ansprüchen 1 oder 5, wobei die verabreichte Dosis von etwa 7,9 mg/kg/Tag bis etwa 21,4 mg/kg/Tag beträgt.

35. Verbindung oder Enantiomer nach den Ansprüchen 1 oder 5, wobei die verabreichte Dosis von etwa 8,6 mg/kg/Tag bis etwa 17,1 mg/kg/Tag beträgt.

36. Verbindung oder Enantiomer nach den Ansprüchen 1 oder 5, wobei die verabreichte Dosis von etwa 400 mg/Tag bis etwa 3000 mg/Tag beträgt.

37. Verbindung oder Enantiomer nach den Ansprüchen 1 oder 5, wobei die verabreichte Dosis von etwa 450 mg/Tag bis etwa 2500 mg/Tag beträgt.

38. Verbindung oder Enantiomer nach den Ansprüchen 1 oder 5, wobei die verabreichte Dosis von etwa 500 mg/Tag bis etwa 2000 mg/Tag beträgt.

39. Verbindung oder Enantiomer nach den Ansprüchen 1 oder 5, wobei die verabreichte Dosis von etwa 550 mg/Tag bis etwa 1500 mg/Tag beträgt.

40. Verbindung oder Enantiomer nach den Ansprüchen 1 oder 5, wobei die verabreichte Dosis von etwa 600 mg/Tag bis etwa 1200 mg/Tag beträgt.

## Revendications

1. Composé, ou sel ou ester pharmaceutiquement acceptable de celui-ci, choisi dans le groupe consistant en la formule (I) et la formule (II): 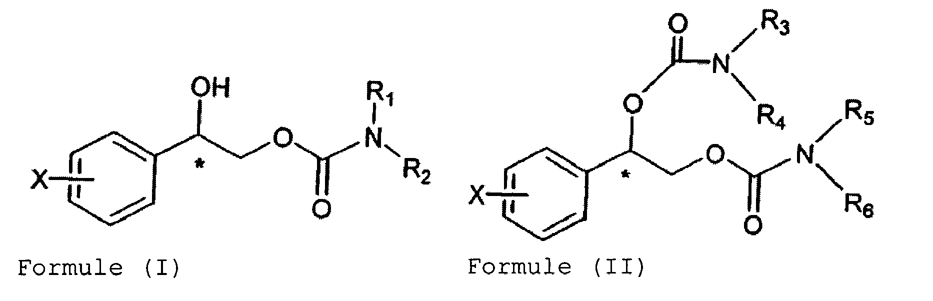 dans lesquelles
le groupe phényle est substitué en X par un à cinq atomes d'halogène choisis dans le groupe consistant en un atome de fluor, un atome de chlore, un atome de brome et un atome d'iode; et
R₁, R₂, R₃, R₄, R₅ et R₆ sont indépendamment choisis dans le groupe consistant en un atome d'hydrogène et un groupe alkyle en C₁ à C₄; où le groupe alkyle en C₁ à C₄ est facultativement substitué par un groupe phényle (où le groupe phényle est facultativement substitué par des substituants indépendamment choisis dans le groupe consistant en un atome d'halogène, un groupe alkyle en C₁ à C₄, un groupe alcoxy en C₁ à C₄, un groupe amino, un groupe nitro et un groupe cyano) pour une utilisation dans le traitement, la prévention, l'inversion, l'arrêt ou l'inhibition d'épileptogenèse chez un sujet humain qui présente un risque de développer une épilepsie ou un trouble lié à une crise mais ne présente pas d'épilepsie ou de preuve clinique de crises.

2. Composé selon la revendication 1, dans lequel X est un atome de chlore.

3. Composé selon la revendication 1, dans lequel X est substitué en position ortho du cycle phényle.

4. Composé selon la revendication 1, dans lequel R₁, R₂, R₃, R₄, R₅ et R₆ sont choisis parmi un atome d'hydrogène.

5. Enantiomère, ou sel ou ester pharmaceutiquement acceptable de celui-ci, choisi dans le groupe consistant en la formule (I) et la formule (II) ou mélange d'énantiomères dans lequel un énantiomère choisi dans le groupe consistant en la formule (I) et la formule (II) prédomine: 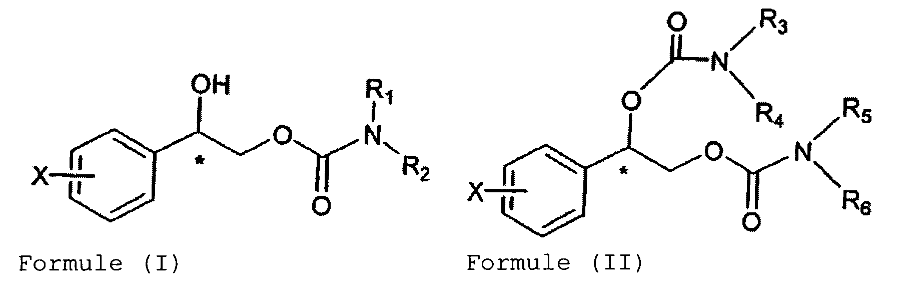 dans lesquelles
le groupe phényle est substitué en X par un à cinq atomes d'halogène choisis dans le groupe consistant en un atome de fluor, un atome de chlore, un atome de brome et un atome d'iode; et
R₁, R₂, R₃, R₄, R₅ et R₆ sont indépendamment choisis dans le groupe consistant en un atome d'hydrogène et un groupe alkyle en C₁ à C₄; où le groupe alkyle en C₁ à C₄ est facultativement substitué par un groupe phényle (où le groupe phényle est facultativement substitué par des substituants indépendamment choisis dans le groupe consistant en un atome d'halogène, un groupe alkyle en C₁ à C₄, un groupe alcoxy en C₁ à C₄, un groupe amino, un groupe nitro et un groupe cyano) pour une utilisation dans le traitement, la prévention, l'inversion, l'arrêt ou l'inhibition d'épileptogenèse chez un sujet humain qui présente un risque de développer une épilepsie ou un trouble lié à une crise mais ne présente pas d'épilepsie ou de preuve clinique de crises.

6. Enantiomère selon la revendication 5, dans lequel X est un atome de chlore.

7. Enantiomère selon la revendication 5, dans lequel X est substitué en position ortho du cycle phényle.

8. Enantiomère selon la revendication 5, dans lequel R₁, R₂, R₃, R₄, R₅ et R₆ sont choisis parmi un atome d'hydrogène.

9. Enantiomère selon la revendication 5, dans lequel un énantiomère choisi dans le groupe consistant en la formule (I) et la formule (II) prédomine jusqu'à environ 90 % ou plus.

10. Enantiomère selon la revendication 5, dans lequel un énantiomère choisi dans le groupe consistant en la formule (I) et la formule (II) prédomine jusqu'à environ 98 % ou plus.

11. Enantiomère selon la revendication 5, dans lequel l'énantiomère choisi dans le groupe consistant en la formule (I) et la formule (II) est un énantiomère choisi dans le groupe consistant en la formule (Ia) et la formule (IIa): 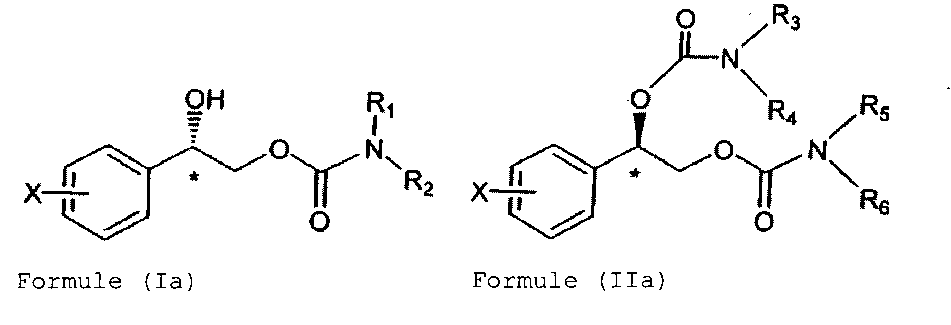 dans lesquelles
le groupe phényle est substitué en X par un à cinq atomes d'halogène choisis dans le groupe consistant en un atome de fluor, un atome de chlore, un atome de brome et un atome d'iode; et
R₁, R₂, R₃, R₄, R₅ et R₆ sont indépendamment choisis dans le groupe consistant en un atome d'hydrogène et un groupe alkyle en C₁ à C₄; où le groupe alkyle en C₁ à C₄ est facultativement substitué par un groupe phényle (où le groupe phényle est facultativement substitué par des substituants indépendamment choisis dans le groupe consistant en un atome d'halogène, un groupe alkyle en C₁ à C₄, un groupe alcoxy en C₁ à C₄, un groupe amino, un groupe nitro et un groupe cyano).

12. Enantiomère selon la revendication 11, dans lequel X est un atome de chlore.

13. Enantiomère selon la revendication 11, dans lequel X est substitué en position ortho du cycle phényle.

14. Enantiomère selon la revendication 11, dans lequel R₁, R₂, R₃, R₄, R₅ et R₆ sont choisis parmi un atome d'hydrogène.

15. Enantiomère selon la revendication 11, dans lequel un énantiomère choisi dans le groupe consistant en la formule (Ia) et la formule (IIa) prédomine jusqu'à environ 90 % ou plus.

16. Enantiomère selon la revendication 11, dans lequel un énantiomère choisi dans le groupe consistant en la formule (Ia) et la formule (IIa) prédomine jusqu'à environ 98 % ou plus.

17. Enantiomère selon la revendication 5, dans lequel l'énantiomère choisi dans le groupe consistant en la formule (I) et la formule (II) est un énantiomère choisi dans le groupe consistant en la formule (Ib) et la formule (IIb) ou un sel ou ester pharmaceutiquement acceptable de celui-ci:

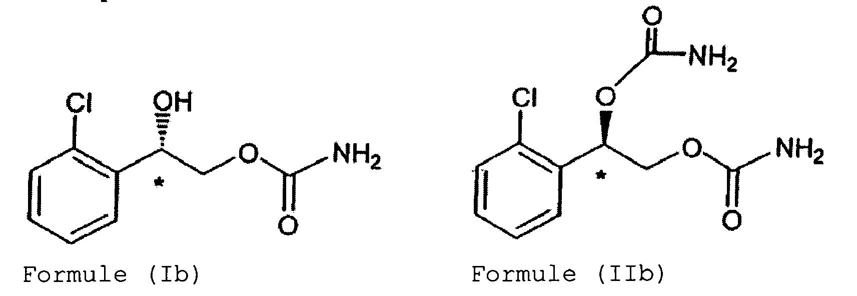

18. Enantiomère selon la revendication 17, dans lequel un énantiomère choisi dans le groupe consistant en la formule (Ib) et la formule (IIb) prédomine jusqu'à environ 90 % ou plus.

19. Enantiomère selon la revendication 17, dans lequel un énantiomère choisi dans le groupe consistant en la formule (Ib) et la formule (IIb) prédomine jusqu'à environ 98 % ou plus.

20. Composé ou énantiomère selon les revendications 1 ou 5, dans lequel le ou les facteurs de prédisposition exposant le patient au risque de développer une épilepsie ou un trouble lié à une crise sont choisis dans le groupe consistant en: une lésion ou un traumatisme de tout type du SNC; des procédures neurochirurgicales, des activités qui présentent un risque de lésion du SNC, par exemple, des activités de lutte, les courses automobiles ou de chevaux et les sports de contact comprenant la boxe; un traumatisme de la moelle épinière; des infections du SNC; l'anoxie, un accident cérébro-vasculaire (AVC); un antécédent d'accidents ischémiques transitoires (TIA); la sténose de la carotide; un antécédent de maladie des vaisseaux athérérosclérotiques, un antécédent d'embolie pulmonaire; un acrosyndrome; des maladies auto-immunes affectant le SNC, par exemple, le lupus; des traumatismes de la naissance; par exemple, une asphyxie périnatale, un arrêt cardiaque, des procédures chirurgicales vasculaires thérapeutiques ou diagnostiques, par exemple, une endartériectomie de la carotide ou une angiographie cérébrale; une hypotension; une lésion du SNC suite à une embolie, une hyperperfusion ou hypoperfusion; une hypoxie; une prédisposition génétique connue à des troubles connus pour répondre aux AEGD; des lésions occupant l'espace du SNC; des tumeurs cérébrales, par exemple, des glioblastomes; un saignement ou une hémorragie dans ou autour du SNC, par exemple, des saignements intracérébraux ou hématomes sous-duraux; un oedème cérébral; des convulsions fébriles; une hyperthermie; une exposition à des agents toxiques ou nocifs; une intoxication ou un sevrage aux médicaments ou drogues, par exemple, la cocaïne, la méthamphétamine ou l'alcool; un antécédent familial de troubles de crise ou un trouble neurologique de type crise liée à l'épilepsie ou un trouble lié à une crise, un antécédent d'état de mal épileptique; un traitement actuel avec des médicaments qui réduisent le seuil de crise, par exemple, le carbonate de lithium, la thorazine ou la clozapine; des preuves de critères de substitution ou biocritères de substitution selon lesquels le patient nécessite un traitement avec un médicament anti-épileptogénique, par exemple, un IRM montrant une sclérose hippocampique, des taux sériques élevés de produits de dégradation neuronale, des taux élevés du facteur neurotrophique ciliaire (CNTF) ou un EEG suggérant un trouble de crise ou d'un trouble neurologique de type crise liée à l'épilepsie.

21. Composé ou énantiomère selon la revendication 20, dans lequel le ou les facteurs de prédisposition exposant le patient au risque de développer une épilepsie ou un trouble lié à une crise sont choisis dans le groupe consistant en: un traumatisme de la tête fermé ou pénétrant; des procédures neurochirurgicales, une sténose de la carotide, un accident cérébro-vasculaire (AVC); un état de mal épileptique et des lésions occupant l'espace du SNC.

22. Composé ou énantiomère selon la revendication 21, dans lequel lesdits facteurs de prédisposition sont un traumatisme de la tête fermé ou un traumatisme de la tête pénétrant ou une procédure neurochirurgicale.

23. Composé ou énantiomère selon la revendication 21, dans lequel lesdits facteurs de prédisposition sont: une attaque, un accident cérébro-vasculaire (AVC), la présence de sténose de la carotide ou d'accident ischémique transitoire.

24. Composé ou énantiomère selon la revendication 23, dans lequel ledit facteur de prédisposition est un état de mal épileptique.

25. Composés ou énantiomères selon les revendications 1 ou 5, dans lesquels lesdits composés (ou énantiomères) ou un sel ou ester pharmaceutiquement acceptable de celui-ci sont administrés en combinaison avec un ou plusieurs autres composés ou agents thérapeutiques.

26. Composés ou énantiomères selon la revendication 25, dans lesquels lesdits un ou plusieurs autres composés ou agents thérapeutiques sont choisis dans le groupe consistant en les composés qui ont une ou plusieurs des propriétés suivantes: une activité antioxydante; un antagonisme du récepteur de NMDA; une capacité à augmenter l'inhibition de GABA endogène; une activité d'inhibiteur de NO synthase; une capacité de liaison au fer, par exemple, un chélateur de fer; une capacité de liaison au calcium, par exemple, un chélateur de Ca (II); une capacité de liaison au zinc, par exemple, un chélateur de Zn (II); la capacité de bloquer les canaux d'ions sodium ou calcium; la capacité d'ouvrir les canaux d'ion potassium ou de chlorure, et des agents thérapeutiques utiles dans le traitement de l'abus de substance.

27. Composés ou énantiomères selon la revendication 25, dans lesquels lesdits un ou plusieurs composés sont choisis dans le groupe consistant en les anti-épileptiques (AED).

28. Composés ou énantiomères selon la revendication 27, dans lesquels ledit anti-épileptique (AED) est choisi dans le groupe consistant en: la carbamazépine, le clobazam, le clonazépam, l'éthosuximide, le felbamate, la gabapentine, la lamotigine, le lévétiracétam, l'oxcarbazépine, le phénobarbital, la phénytoïne, la prégabaline, la primidone, la rétigabine, le talampanel, la tiagabine, le topiramate, le valproate, la vigabatrine, le zonisamide, les benzodiazépines, les barbiturates ou les hypnotiques sédatifs.

29. Composé ou énantiomère selon les revendications 1 ou 5, dans lequel la quantité thérapeutiquement efficace va d'environ 5,7 mg/kg/jour à environ 42,9 mg/kg/jour.

30. Composé ou énantiomère selon les revendications 1 ou 5, dans lequel ladite quantité thérapeutique est progressivement diminuée lorsque le traitement du processus épileptogénique progresse chez ledit patient.

31. Composé ou énantiomère selon les revendications 25, 26, 27 ou 28, dans lequel la quantité desdits un ou plusieurs autres composés ou agents thérapeutiques administrés en combinaison avec ledit composé (ou énantiomère) ou un sel ou ester pharmaceutiquement acceptable de celui-ci est progressivement diminuée lorsque le traitement du processus épileptogénique progresse chez ledit patient.

32. Composé ou énantiomère selon les revendications 1 ou 5, dans lequel la dose administrée va d'environ 6,4 mg/kg/jour à environ 35,7 mg/kg/jour.

33. Composé ou énantiomère selon les revendications 1 ou 5, dans lequel la dose administrée va d'environ 7,1 mg/kg/jour à environ 28,6 mg/kg/jour.

34. Composé ou énantiomère selon les revendications 1 ou 5, dans lequel la dose administrée va d'environ 7,9 mg/kg/jour à environ 21,4 mg/kg/jour.

35. Composé ou énantiomère selon les revendications 1 ou 5, dans lequel la dose administrée va d'environ 8,6 mg/kg/jour à environ 17,1 mg/kg/jour.

36. Composé ou énantiomère selon les revendications 1 ou 5, dans lequel la dose administrée va d'environ 400 mg/jour à environ 3 000 mg/jour.

37. Composé ou énantiomère selon les revendications 1 ou 5, dans lequel la dose administrée va d'environ 450 mg/jour à environ 2 500 mg/jour.

38. Composé ou énantiomère selon les revendications 1 ou 5, dans lequel la dose administrée va d'environ 500 mg/jour à environ 2 000 mg/jour.

39. Composé ou énantiomère selon les revendications 1 ou 5, dans lequel la dose administrée va d'environ 550 mg/jour à environ 1 500 mg/jour.

40. Composé ou énantiomère selon les revendications 1 ou 5, dans lequel la dose administrée va d'environ 600 mg/jour à environ 1 200 mg/jour.
